# EUROPEAN PATENT APPLICATION

(11) **EP 4 556 457 A2**
(43) Date of publication of application: **21.05.2025**
(21) Application number: 25154981.2
(22) Date of filing: 15.06.2017
(51) Int. Cl.: C07C 39/23

(54) **METHODS AND COMPOSITIONS FOR POTENTIATING STEM CELL THERAPIES**

(30) Priority: 15.06.2016 US 201662350708 P
(62) Divisional of application: 17814138.8
(71) Applicant: Ojai Energetics PBC, Ojai, CA 93023 (US)
(72) Inventor: KLEIDON, William, Ojai, 93023 (US)
(74) Representative: Mewburn Ellis LLP

(57) **Abstract**

The present disclosure relates to cannabinoid compositions used in combination with stem cell therapies. These compositions can be encapsulated (e.g., microencapsulated). In particular, these compositions can be administered to a subject, such as through oral consumption or topical treatment.

## Description

### CROSS-REFERENCE

This application claims the benefit of U.S. Provisional Application No. 62/350,708, filed June 15, 2016, which is entirely incorporated herein by reference.

### BACKGROUND

Stem cells are responsible both for generation of tissue during development and for regeneration following tissue damage. Stem cells exhibit two key properties, the ability to self-renew and the ability to give rise to more specialized cell types.

Research into stem cells has shown promise for treating many diseases. One of the most established forms of stem cell therapy is bone marrow transplant, in which the hematopoietic stem cells of a patient are replaced with donor stem cells. The donor stem cells populate the bone marrow of the patient and regenerate the hematopoietic system. Another type of stem cell therapy that is well established is use of epithelial stem cells in treating large skin wounds such as those of burn patients. Cell cultured epithelial autografts allow small grafts from unaffected skin to be expanded in culture allowing coverage of extensive wounds. Stem cell therapies have also had success in treatment of arthritis, autoimmune diseases, vision and hearing loss, diabetes and orthopedics. Use of stem cells in neurological diseases is an area of active research and promising results have been seen in disease models of stroke and multiple sclerosis.

Many challenges remain for stem cell therapies. Use of autologous adult stem cells is constrained by their limited replicative potential. Use of embryonic stem cells can alleviate this concern but donor stem cells must be carefully matched to minimize immune rejection and immunosuppressive drugs may still be required. Integration remains a challenge as stem cell derived tissue needs to be able to function together with existing structures. Control of differentiation and replication is important as stem cells also have the ability to foster undesired growth.

### SUMMARY

There is a considerable need for methods and compositions that can potentiate the function of stem cell therapies. The inventions described in the present disclosure address this need and provide other related advantages as well.

An aspect of the present disclosure provides a method for potentiating function of a stem cell in a subject, comprising (a) administering to said subject said stem cell; (b) administering to said subject a pharmaceutical composition comprising an encapsulated cannabinoid compound, wherein (b) takes place prior to, concurrent with, or subsequent to step (a). In some embodiments, step (b) further comprises at least one terpene compound. In some embodiments, the treatment with step (b) reduces the side effects usually associated with step (a). In some embodiments, steps (a) and (b) exhibit a synergetic effect on a process in the subject.

In some embodiments, said subject suffers or is suspected of suffering from a disease selected from the group of acute leukemia, chronic leukemia, and lymphoma, an inherited platelet abnormality, a plasma cell disorder, an autoimmune disease, a myeloproliferative disorder, a lymphoproliferative disorder, a phagocyte disorder, myelodysplastic syndrome, a histiocytic disorder, a congenital immune system disorder, Parkinson's disease, amyotrophic lateral sclerosis, Alzheimer's disease, and multiple sclerosis, stroke, diabetes, infertility, vision loss or other eye diseases, a lysosomal storage disease, peripheral arterial diseases, ischemic limb injury, diabetes, heart disease, liver disease, bone disease, muscular dystrophy, dental disease, and cancer.

In some embodiments, said subject suffers from an injury selected from the group of spine and spinal cord injuries, wounds, thermal or chemical burns, sports injuries, occupational injuries, or a brain injury.

In some embodiments, said stem cell is from stem cells that are induced pluripotent stem cells, embryonic stem cells, fetal stem cells, or adult stem cells.

In some embodiments, said stem cell expresses at least one of AA4, AA4.1, P-gp (CD243), ABCB5, ABCG2 (CDw338), ALDH, alkaline phosphatase, alpha6-integrin, WNT2B, antithrombin III (AT), asialo GM1, Bcl-2, beta1-integrin, bromodeoxyuridine, c-kit (CD117), c-Met, C1qR(p), END (CD105), PROM1 (CD133) , ALCAM (CD166), ITGB1 (CD29), TNFRSF8 (CD30), PECAM-1 (CD31), Siglec-3 (CD33), CD34, CD44, NCAM (CD56), CD73, CD9, CD90, CDCP1, Circulating anticoagulants protein C (PC), CK19, CLV3, cyclic CMP, ECMA-7, EDR1, EEC, FGF-4, Flk-2, Flk1(+), Flt3/Flk2, FMS (CD115), FORSE-1, G alpha16, GDF3, GFPM, Gli2, Gli3, glial fibrillary acidic protein, glycoprotein IB, GSTA1, HAS2 gene expression, Her5, hMYADM, HSA, hsp25, Id2, IL-3Ralpha, Integrins, interleukin-3 receptor alpha chain, Iron oxide nanoparticles, KDR, Keratin 15 (aka. CK15, Cytokeratin 15) , Keratin 19 (aka. CK19, Cytokeratin 19, K19), Kit, L-selectin (CD62L), Lamin A/C, Lewis X antigen (Le(X)), LeX, Lgr5, Lrp4, MCM2, MCSP, Metallothionein (MT) crypt-restricted immunopositivity indices (MTCRII), monosomy 7, Mouse orthologue of ARX, MRP4, Msi-1, Musashi, Musashi-1, Mutant BCRP, nestin, neurofilament microtubule-associated protein 2, neuron-glial antigen 2 (NG2), notch 1, nrp-1, Nucleostemin, OC.3, Oct-4, OST-PTP, P-gp/MDR1, p21, p63, p75, PCLP, PCNA, PECAM, PgP-1, phosphorylating-p38, Podocalyxin, procalcitonin (PCT), PSCs, pSV2gpt, PTPRC, purified LRC, Rat liver fatty acid-binding protein/human growth hormone transgenes (Fabpl/hGH), RC1 antigen, Rex-1, Sca-1, SCF, Sialyl-lactotetra, Side Population (SP), SOX10, SOX2, SOX9, SP phenotype, SSEA-1, SSEA-3, SSEA-4, Stat3, Stat5, Stella, Stra8, Stro-1, Tartrate-resistant acid phosphatase (TRAcP), TdT, telomerase reverse transcriptase, electrophoretic pattern of hemoglobin, Thrombomucin, Thy-1, Tra-1-60, TWIST1, VEGFR-2, vimentin, X-Smoothened, XKrk1, or Zac1.

In some embodiments, said stem cell is from stem cells that exhibit totipotency, pluripotency, bipotency or monopotency.

In some embodiments, said stem cell is from stem cells that yield fibroblasts, keratinocytes, melanocytes, cold-sensitive primary sensory neurons, auditory inner hair cells or organ of Corti cells, Merkel cells, photoreceptor cells, taste bud cells, cholinergic neural cells, adrenergic neural cells, peptidergic neural cells, hepatocytes, adipocytes, liver lipocytes, kidney glomerulus podocytes, pancreatic duct cells, gall bladder epithelial cells, pericytes, corneal fibroblasts, skeletal muscle cells, cardiac muscle cells, Purkinje fiber cells, erythrocytes, megakaryocytes, monocytes, Langerhans cells, osteoclasts, osteoblasts, dendritic cells, microglial cells, neutrophil granulocyte, hybridoma cells, mast cells, helper T cells, suppressor T cells, cytotoxic T cells, natural killer T cells, B cells, oocytes, spermatids, ovarian follicle cells, Schwann cells, satellite glial cells, enteric glial cells, astrocytes, neuron cells, oligodendrocytes, anterior lens epithelial cells, crystallin-containing lens fiber cells, somatotropes, Corticotropes, melanotropes, thyroid gland cells, or odontocytes.

In some embodiments, said stem cell is from stem cells that are delivered via intravenous infusion, intradermal, transplanted microvascular bed of bone marrow, subcutaneous, oral (e.g., ingestion or inhalation), transdermal (topical), transmucosal, rectal administration, engineered monolayer tissue transplantation, intraarterially, intramuscularly, intratracheally, intraperitoneally, intravitreally, or via direct injection to a target site.

In some embodiments, said pharmaceutical composition comprises nanocapsules, wherein said nanocapsules comprise an individual nanocapsule comprising said encapsulated cannabinoid compound.

In some embodiments, said nanocapsules increase stem cell growth. In some embodiments, the nanocapsules are administered following said stem cell therapy.

In some embodiments, said nanocapsules are administered to said subject by inhalation. In some embodiments, the nanocapsules are vaporized. In some embodiments, said nanocapsules are nebulized. In some embodiments, said nanocapsules are administered orally. In some embodiments, the nanocapsules are incorporated into a food or beverage. In some embodiments, the nanocapsules are administered topically.

In some embodiments, said nanocapsules are water soluble.

In some embodiments, said at least one terpene compound is derived from quillaja *(Quillaja saponaria).* In some embodiments, said cannabinoid compound comprises cannabidiol (CBD). In some embodiments, the cannabinoid compound comprises 0.3% tetrahydrocannabinol (THC) or less. In some embodiments, the nanocapsules are water soluble.

In some embodiments, said subject suffers or is suspected of suffering from a disease or injury, and wherein, subsequent to (b), said subject is monitored for a progress of said disease or injury in response to said subject being administered said pharmaceutical composition.

In some embodiments, said subject suffers or is suspected of suffering from said disease, and wherein, subsequent to (b), said subject is monitored for a progression or regression of said disease in response to said subject being administered said pharmaceutical composition

In another aspect, the present disclosure provides a kit comprising a composition comprising an effective amount of an encapsulated cannabinoid compound; and instructions for administering to a subject, undergoing a stem cell therapy, a therapeutically effective amount of said food composition.

In some embodiments, said composition is a food composition. In some embodiments, said composition is a pharmaceutical composition. In some embodiments, said composition comprises nanocapsules, wherein said nanocapsules comprise an individual nanocapsule comprising said encapsulated cannabinoid compound. In some embodiments, the nanocapsule delivery device is a nebulizer. In some embodiments, the nanocapsule delivery device is a vaporizer.

In some embodiments, the nanocapsules include a terpene compound. In some embodiments, the terpene compound is derived from quillaja *(Quillaja saponaria).*

Other goals and advantages of the invention will be further appreciated and understood when considered in conjunction with the following description and accompanying drawings. While the following description may contain specific details describing particular embodiments of the invention, this should not be construed as limitations to the scope of the invention but rather as an exemplification of preferable embodiments. For each aspect of the invention, many variations are possible as suggested herein that are known to those of ordinary skill in the art. A variety of changes and modifications can be made within the scope of the invention without departing from the spirit thereof.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### DETAILED DESCRIPTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein can be employed in practicing the invention. It is intended that the following claims define the scope of the invention and that methods and structures within the scope of these claims and their equivalents be covered thereby.

The term "about" or "nearly" as used herein refers to within +/- 10%, 9%, 8%, 7%, 6%, 5%, 4%, 3%, 2%, or 1% of the designated amount.

"Treatment", "treating", "palliating" and "ameliorating", as used herein, are used interchangeably. These terms refer to an approach for obtaining beneficial or desired results including but not limited to therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder.

The term "migration potential" as used herein, can refer to the migratory ability of therapeutic cells to migrate to lesion sites. The term "migration potential" can refer to the migratory ability of a stem cell under culture conditions.

This disclosure provides a method for potentiating function of a stem cell in a subject, comprising: (a) administering to said subject the stem cell; (b) administering to said subject a pharmaceutical composition comprising a encapsulated cannabinoid compound, wherein (b) takes place prior to, concurrent with, or subsequent to step (a).

### Stem cells

The stem cells used in this disclosure can be induced pluripotent stem cells, embryonic stem cells, fetal stem cells, or adult stem cells. Fetal stem cells can be either fetal proper stem cells derived from the fetus itself or extraembryonic fetal stem cells.

The stem cells used in this disclosure can be Induced Pluripotent Stem Cells (iPSCs) which are artificially derived stem cells from a non-pluripotent cell, typically an adult somatic cell, produced by inducing expression of one or more stem cell specific genes. Such stem cell specific genes include, but are not limited to, the family of octamer transcription factors, i.e. Oct-3, Oct-4; the family of Sox genes, i.e. Sox1, Sox2, Sox3, Sox 15 and Sox 18; the family of Klf genes, i.e. Klf1, Klf2, Klf4 and Klf5; the family of Myc genes, i.e. c-myc and L-myc; the family of Nanog genes, i.e. OCT4, NANOG and REX1; or LIN28. Examples of iPSCs are described in Takahashi K. et al. (2007) Cell 131:861-72; Takahashi K. & Yamanaka S. (2006) Cell 126: 663-76; Okita K. et al. (2007) Nature 448:260-262; Yu, J. et al. (2007) Science 318:1917-20; and Nakagawa, M. et al. (2008) Nat. Biotechnol. 26:101-106.

Stem cells utilized in the compositions and methods disclosed herein can be derived in a variety of ways. For example, the stem cells can be isolated from the tissue which they are being used to treat, or from other tissues. In some instances, the stem cells are isolated from blood, brain, bone marrow, blood vessels, skeletal muscle, skin, teeth, heart, gut, or liver tissue. The stem cells can be isolated from the subject, from a relative of the subject, from a donor, from a tissue bank or from a stem cell bank.

The stem cells used in this disclosure can be isolated from blood (i.e., hematopoietic tissue). Possible sources of human hematopoietic tissue include, but are not limited to, embryonic hematopoietic tissue, fetal hematopoietic tissue, and post-natal hematopoietic tissue. Embryonic hematopoietic tissue can be yolk sac or embryonic liver. Fetal hematopoietic can be selected from fetal liver, fetal bone marrow and fetal peripheral blood. The post-natal hematopoietic can be cord blood, bone marrow, normal peripheral blood, mobilized peripheral blood, hepatic hematopoietic tissue, or splenic hematopoietic tissue.

The stem cells utilized may express any stem cell markers. Many stem cell markers are known in the art, some examples of stem cell markers that may be expressed include but are not limited to AA4, AA4.1, P-gp (CD243), ABCB5, ABCG2 (CDw338), ALDH, alkaline phosphatase, alpha6-integrin, WNT2B, antithrombin III (AT), asialo GM1, Bcl-2, beta1-integrin, bromodeoxyuridine, c-kit (CD117), c-Met, C1qR(p), END (CD105), PROM1 (CD133), ALCAM (CD166), ITGB1 (CD29), TNFRSF8 (CD30), PECAM-1 (CD31), Siglec-3 (CD33), CD34, CD44, NCAM (CD56), CD73, CD9, CD90, CDCP1, Circulating anticoagulants protein C (PC), CK19, CLV3, cyclic CMP, ECMA-7, EDR1, EEC, FGF-4, Flk-2, Flk1(+), Flt3/Flk2, FMS (CD115), FORSE-1, G alpha16, GDF3, GFPM, Gli2, Gli3, glial fibrillary acidic protein, glycoprotein IB, GSTA1, HAS2 gene expression, Her5, hMYADM, HSA, hsp25, Id2, IL-3Ralpha, Integrins, interleukin-3 receptor alpha chain, Iron oxide nanoparticles, KDR, Keratin 15 (aka. CK15, Cytokeratin 15) , Keratin 19 (aka. CK19, Cytokeratin 19, K19), Kit, L-selectin (CD62L), Lamin A/C, Lewis X antigen (Le(X)), LeX, Lgr5, Lrp4, MCM2, MCSP, Metallothionein (MT) crypt-restricted immunopositivity indices (MTCRII), monosomy 7, Mouse orthologue of ARX, MRP4, Msi-1, Musashi, Musashi-1, Mutant BCRP, nestin, neurofilament microtubule-associated protein 2, neuron-glial antigen 2 (NG2), notch 1, nrp-1, Nucleostemin, OC.3, Oct-4, OST-PTP, P-gp/MDR1, p21, p63, p75, PCLP, PCNA, PECAM, PgP-1, phosphorylating-p38, Podocalyxin, procalcitonin (PCT), PSCs, pSV2gpt, PTPRC, purified LRC, Rat liver fatty acid-binding protein/human growth hormone transgenes (Fabpl/hGH), RC1 antigen, Rex-1, Sca-1, SCF, Sialyl-lactotetra, Side Population (SP), SOX10, SOX2, SOX9, SP phenotype, SSEA-1, SSEA-3, SSEA-4, Stat3, Stat5, Stella, Stra8, Stro-1, Tartrate-resistant acid phosphatase (TRAcP), TdT, telomerase reverse transcriptase, electrophoretic pattern of hemoglobin, Thrombomucin, Thy-1, Tra-1-60, TWIST1, VEGFR-2, vimentin, X-Smoothened, XKrk1, and Zac1.

The stem cells may express any single stem cell marker. Alternatively the stem cells may express several stem cell markers. In some cases, the stem cells may express at least 2, 3, 4, 5, 6, 7, 8, 9, 10, 20, or more stem cell markers.

In some instances, the stem cells may express a cannabinoid receptor or a putative cannabinoid receptor. The stem cells may express a cannabinoid receptor type 1, a cannabinoid receptor type 2, or another cannabinoid receptor. Examples of other cannabinoid receptors include: GPR18, GPR55, and GPR119.

The stem cells used in this disclosure can be maintained in culture before administration to the subject by any method known in the art. The stem cells can be maintained with or without a feeder layer, and in serum containing or serum free culture media. The stem cells can be maintained in conditioned media.

The stem cells can be partially differentiated before administration to the subject. Many methods for differentiating stem cells towards either mixed cell populations or specific cell lineages are known in the art. For example, embryonic stem cells or iPSCs can be cultured under conditions to allow formation of embryoid bodies thus triggering differentiation. In another example the embryonic stem cells or iPSCs can be differentiated on a layer of stromal cells. In another example the embryonic stem cells or iPSCs can be differentiated on extracellular matrix proteins.

The presence of growth factors, serum or small molecules in the media can influence the differentiation of the embryonic stem cells, or iPSCs. These growth factors include but are not limited to Bone morphogenetic proteins, Colony-stimulating factors, Epidermal growth factor, Ephrins, Fibroblast growth factor, Foetal Bovine Somatotrophin, GDNF family of ligands, Hepatocyte growth factor, Insulin-like growth factor, Interleukins, Keratinocyte growth factor, Migration-stimulating factor, Macrophage-stimulating protein, Myostatin, Neuregulins, Neurotrophins, Placental growth factor, Platelet-derived growth factor, Transforming growth factors, Tumor necrosis factor-alpha, Vascular endothelial growth factor and Wnt signaling pathway proteins.

For example, culturing murine embryonic stem cells in the presence of VEGF, Activin, BMP4, StemPro34, Glutamine, Ascorbic acid, bFGF and FGF10 can induce differentiation of cardiomyocytes, (Kokkinopoulos, Ioannis, et al. "Cardiomyocyte differentiation from mouse embryonic stem cells using a simple and defined protocol." Developmental Dynamics 245.2 (2016): 157-165.). In another example human iPSCs can be grown with SCF, TPO, FLT3L, VEGF, IL-3, IL-6, and EPO to induce differentiation to erythroid cells, (Dorn, Isabel, et al. "Erythroid differentiation of human induced pluripotent stem cells is independent of donor cell type of origin." haematologica 100.1 (2015): 32-41.).

The stem cells used in the disclosure described herein can be able to differentiate into any number of cell types. For example, stem cells used can be totipotent, pluripotent, bipotent or unipotent. In some preferred applications the stem cells used are pluripotent or bipotent.

The stem cells used in this disclosure can be capable of differentiating into cells of one or more of the ectodermal, mesodermal or endodermal lineages. Examples of cells that differentiate from the ectodermal lineage include, but are not limited to epidermal cells, neurogenic cells, and neurogliagenic cells. Examples of cells that differentiate from the mesodermal lineage or give rise to specific mesodermal cells include, but are not limited to, cells that are adipogenic, leiomyogenic, chondrogenic, cardiogenic, dermatogenic, hematopoetic, hemangiogenic, myogenic, nephrogenic, urogenitogenic, osteogenic, pericardiogenic, or stromal. Examples of cells that differentiate from the endodermal lineage include, but are not limited to pleurogenic cells, and hepatogenic cells, cell that give rise to the lining of the intestine, and cells that give rise to pancreogenic and splanchogenic cells.

The stem cells used in this disclosure can be able to differentiate into any type of mature cell or precursor. Selected examples of types of cells include, autonomic neuron cells, blood and immune system cells, central nervous system neurons and glial cells, contractile cells, exocrine secretory epithelial cells, extracellular matrix cells, germ cells, heart muscle cells, hormone-secreting cells, integumentary system, interstitial cells, keratinizing epithelial cells, lens cells, metabolism and storage cells, nervous system, nurse cell, sense organ and peripheral neuron supporting cells, sensory transducer cells, skeletal muscle cell, and wet stratified barrier epithelial cells.

The stem cells used in this disclosure can be able to differentiate into any mature cell or precursor. Selected examples of cells include, but are not limited to, salivary gland mucous cells (polysaccharide-rich secretion), salivary gland number 1 cells (glycoprotein enzyme-rich secretion), von Ebner's gland cells in tongue (washes taste buds), mammary gland cells (milk secretion), lacrimal gland cells (tear secretion), ceruminous gland cells in ear (earwax secretion), eccrine sweat gland dark cells (glycoprotein secretion), eccrine sweat gland clear cells (small molecule secretion), apocrine sweat gland cells (odoriferous secretion, sex-hormone sensitive), gland of moll cells in eyelid (specialized sweat gland), sebaceous gland cells (lipid-rich sebum secretion), Bowman's gland cells
in nose (washes olfactory epithelium), Brunner's gland cells in duodenum (enzymes and alkaline mucus), seminal vesicle cells (secretes seminal fluid components, including fructose for swimming sperm), prostate gland cells (secretes seminal fluid components), bulbourethral gland cells (mucus secretion), Bartholin's gland cells (vaginal lubricant secretion), gland of Littre cells (mucus secretion), uterus endometrium cells (carbohydrate secretion),
goblet cells of respiratory and digestive tracts (mucus secretion), stomach lining mucous cells (mucus secretion), gastric gland zymogenic cells (pepsinogen secretion), gastric gland oxyntic cells (hydrochloric acid secretion), pancreatic acinar cells (bicarbonate and digestive enzyme secretion, paneth cells of small intestine (lysozyme secretion), type ii pneumocytes of lung (surfactant secretion), Clara cells of lung, somatotropes, lactotropes, thyrotropes, gonadotropes, corticotropes, intermediate pituitary cells, magnocellular neurosecretory cells, gut and respiratory tract cells, thyroid gland cells, thyroid epithelial cell, parafollicular cell, parathyroid gland cells, parathyroid chief cells, oxyphil cells, adrenal gland cells, chromaffin cells, leydig cells of testes secreting testosterone, theca interna cells of ovarian follicle secreting estrogen, corpus luteum cells of ruptured ovarian follicle secreting progesterone, granulosa lutein cells, theca lutein cells, juxtaglomerular cells (renin secretion), macula densa cells of kidney, peripolar cells of kidney, mesangial cells of kidney, epidermal keratinocytes (differentiating epidermal cell), epidermal basal cells (stem cell), keratinocytes of fingernails and toenails, nail bed basal cells (stem cell), melanocytes, medullary hair shaft cells, Cortical hair shaft cells, cuticular hair shaft cells, cuticular hair root sheath cells, hair root sheath cells of Huxley's layer, hair root sheath cells of Henle's layer, external hair root sheath cells, hair matrix cells (stem cell), surface epithelial cells of stratified squamous epithelium of cornea, tongue, oral cavity, esophagus, anal canal,
distal urethra and vagina, basal cells (stem cell) of epithelia of cornea, tongue, oral cavity, esophagus, anal canal, distal urethra and vagina, urinary epithelium cells (lining urinary bladder and urinary ducts), auditory inner hair cells of organ of Corti, auditory outer hair cells of organ of Corti, basal cells of olfactory epithelium (stem cell for olfactory neurons), cold-sensitive primary sensory neurons, heat-sensitive primary sensory neurons, Merkel cell of epidermis (touch sensor), olfactory receptor neurons, pain-sensitive primary sensory neurons (various types), photoreceptor cells of retina in eye:, photoreceptor rod cells, photoreceptor blue-sensitive cone cell of eye, photoreceptor green-sensitive cone cell of eye, photoreceptor red-sensitive cone cell of eye, proprioceptive primary sensory neurons (various types), touch-sensitive primary sensory neurons (various types), type i carotid body cells (blood ph sensor), type ii carotid body cells (blood ph sensor), type i hair cells of vestibular system of ear (acceleration and gravity), type ii hair cells of vestibular system of ear (acceleration and gravity), type i taste bud cells, cholinergic neural cells (various types), adrenergic neural cells (various types), peptidergic neural cells (various types), inner pillar cells of organ of Corti, outer pillar cells of organ of Corti, inner phalangeal cells of organ of Corti, outer phalangeal cells of organ of Corti, border cells of organ of Corti, hensen cells of organ of Corti, vestibular apparatus supporting cells, taste bud supporting cells, olfactory epithelium supporting cells, schwann cells, satellite glial cells (encapsulating peripheral nerve cells bodies), enteric glial cells, astrocyte (various types), neuron cellss (large variety of types, still poorly classified), oligodendrocytes, spindle neurons, anterior lens epithelial cells, crystallin-containing lens fiber cells, hepatocytes (liver cells), adipocytes (white fat cells and brown fat cells), liver lipocytes, kidney parietal cells, kidney glomerulus podocytes, kidney proximal tubule brush border cells, loop of henle thin segment cells, kidney distal tubule cells, kidney collecting duct cells, principal cells, intercalated cells, type i pneumocytes (lining air space of lung cell), pancreatic duct cells (centroacinar cell), nonstriated duct cells (of sweat gland, salivary gland, mammary gland, etc.), principal cells, intercalated cells, duct cells (of seminal vesicle, prostate gland, etc.), intestinal brush border cells (with microvilli), exocrine gland striated duct cells, gall bladder epithelial cells, ductulus efferens nonciliated cells, epididymal principal cells, epididymal basal cells, ameloblast epithelial celsl (tooth enamel secretion), planum semilunatum epithelial cells of vestibular system of ear (proteoglycan secretion), organ of Corti interdental epithelial cells (secreting tectorial membrane covering hair cells), loose connective tissue fibroblasts, corneal fibroblasts (corneal keratocytes), tendon fibroblasts, bone marrow reticular tissue fibroblasts, other nonepithelial fibroblasts, pericytes, nucleus pulposus cells of intervertebral disc, cementoblast/cementocytes (tooth root bonelike ewan cell secretion), odontoblast/odontocytes (tooth dentin secretion), hyaline cartilage chondrocytes,
fibrocartilage chondrocytes, elastic cartilage chondrocytes, osteoblast/osteocytes, osteoprogenitor cells (stem cell of osteoblasts), hyalocyte of vitreous body of eye, stellate cells of perilymphatic space of ear, hepatic stellate cells (ito cell), pancreatic stelle cells, red skeletal muscle cells (slow), white skeletal muscle cells (fast), intermediate skeletal muscle cells, nuclear bag cell of muscle spindle, nuclear chain cell of muscle spindle, satellite cells (stem cell), ordinary heart muscle cells, nodal heart muscle cells, purkinje fiber cells, smooth muscle cells (various types), myoepithelial cell of iris, myoepithelial cell of exocrine glands, erythrocytes (red blood cell), megakaryocytes (platelet pecursor), monocytes (white blood cell ), connective tissue macrophages (various types), epidermal langerhans cells, osteoclasts (in bone), dendritic cells (in lymphoid tissues), microglial cells (in central nervous system), neutrophil granulocytes, eosinophil granulocytes, basophil granulocytes, hybridoma cells, mast cells, helper t cells, suppressor t cells, cytotoxic t cell, natural killer t cells, b cells, natural killer cells, reticulocytes, stem cells and committed progenitors for the blood and immune system (various types), oogonium/oocytes, spermatids, spermatocytes, spermatogonium cells (stem cell for spermatocyte), spermatozoon, ovarian follicle cells, sertoli cells (in testis), thymus epithelial cells, and interstitial kidney cells.

The stem cells used can be a single type of stem cell, a mixed population of stem cells or two or more separate populations of stem cells. The stem cells used can be derived from a single source or from multiple sources. The stem cells may differentiate into a single cell type or into several different cell types.

The stem cells used in this disclosure may have been genetically modified. For example, iPSCs generated from autologous cells can be modified to correct a deficit or mutation. Modification methods may include CRISPR, zinc finger nucleases, Talens, homologous recombination, transfection or transduction. The modification can be used to shut down a gene, replace a faulty gene, or increase expression of a gene product.

The stem cells of this disclosure can be delivered by any method in the art. Methods that are known in the art include parenteral, e.g., intravenous infusion, intradermal, transplanted microvascular bed of bone marrow, subcutaneous, oral (e.g., ingestion or inhalation), transdermal (topical), transmucosal, rectal administration, or engineered monolayer tissue transplantation. In certain particular aspects, the population of modified cells is administered from a route selected from a group consisting of: intraarterially, intramuscularly, subcutaneously, transdermally, intratracheally, intraperitoneally, intravitreally, intranasally, via direct injection to a target site such as into bone compartments, into the heart or into spinal fluid. In certain aspects, the population of modified cells is administered in or proximal to a site of injury. In some embodiments the cells can be administered by multiple routes and sites either simultaneously or sequentially.

The route of delivery of the stem cells will depend on the application. For example, stem cells can be delivered topically for treatment of skin. Stem cells can be delivered intravenously for treatment of a blood disease. Stem cells can be delivered intramuscularly for treatment of a muscle disease. Stem cells can be delivered into the brain via intranasal delivery for treatment of a brain disease. Stem cells can be delivered into the heart via intramyocardial injection for treatment heart disease. Stem cells may also be delivered intravenously for treatment of a number of non-blood-related diseases and disorders.

In some embodiments the stem cells can be administered with a scaffolding support. Scaffolding supports that can be used include bioactive glass, hydrogels, photopolymerizable poly(ethene) glycol hydrogels, collagen gel, matrigel (Corning), or decellularized tissue such as cartilage or connective tissue. The stem cells can be added to the scaffold after it is formed, or the scaffold can be formed around the stem cells.

The stem cells of the disclosure described herein can be used to treat a disease. Examples of types of diseases that can be treated include blood diseases, cancer, immune diseases, cardiovascular diseases, muscle diseases, metabolic diseases, neurological diseases, connective tissue diseases, eye diseases. The stem cells can be used to replace cells that have been lost and/or damaged by disease. The stem cells can be used to replace cells that carry deleterious mutations.

Some examples of blood diseases that can be treated with a stem cell therapy include, but are not limited to, acute leukemia, chronic leukemia, lymphoma, inherited platelet abnormality, plasma cell disorders, autoimmune diseases such as Lupus, myeloproliferative disorders, lymphoproliferative disorders, phagocyte disorders, myelodysplastic syndrome, a histiocytic disorder, or a congenital immune system disorder. For example, leukemia can be treated by using cytotoxic drugs and/or radiation therapy to kill the cancerous and noncancerous blood stem cells, and then replacing the stem cells with donor bone marrow. The stem cells in the donor bone marrow repopulate the hematopoietic system. Stem cell treatments for autoimmune diseases can involve killing the mature autoreactive immune cells with cytotoxic drugs and/or radiation and then repopulating the hematopoietic system with previously isolated autologous blood stem cells.

Examples of neurological diseases that can be treated with the methods of the disclosure include, but are not limited to, Parkinson's disease, Amyotrophic lateral sclerosis, Alzheimer's disease, multiple sclerosis, and stroke. These diseases are associated with loss of neurons and/or glial cells and stem cell therapies may be able to replace these cells.

Treatment of cardiovascular and muscle diseases involve using stem cells to replace lost or damaged muscle fibers. Human embryonic stem cell derived cardiomyocytes have been shown to successfully integrate into guinea pig hearts, (Shiba, Yuji, et al. "Human ES-cell-derived cardiomyocytes electrically couple and suppress arrhythmias in injured hearts." Nature 489.7415 (2012): 322-325.).

Other examples of diseases that can be treated with the methods of the disclosure include, but are not limited to, diabetes, infertility, lysosomal storage diseases, peripheral arterial diseases, diabetes, heart disease, liver disease, bone disease, muscular dystrophy, dental disease, or cancer.

Stem cell therapy has been successfully used to regrow teeth in mice, (Ohazama, A., et al. "Stem-cell-based tissue engineering of murine teeth." Journal of Dental Research 83.7 (2004): 518-522.). Stem cell therapy in combination with gene editing has been used to restore hearing in deaf chickens, (Izumikawa, Masahiko, et al. "Auditory hair cell replacement and hearing improvement by Atoh1 gene therapy in deaf mammals." Nature medicine 11.3 (2005): 271-276.). Delivering retinal stem cells to the eye can treat blindness caused by macular degeneration. Embryonic stem cells can be differentiated into insulin producing beta cells which can be delivered to the pancreas to treat diabetes.

The methods of the disclosure described herein can also be used to treat injuries. Injuries that may potentially be treated with stem cells include spine and spinal cord injuries, wounds, thermal or chemical burns, sports injuries, occupational injuries, or a brain injury. In adults wounds are frequently healed through the formation of scar tissue, placing stem cells in the wound may allow regeneration of tissue and facilitate healing without scar tissue formation. Stem cells can be used in spinal injuries to promote healing and locomotor recovery, (Cummings, Brian J., et al. "Human neural stem cells differentiate and promote locomotor recovery in spinal cord-injured mice." Proceedings of the National Academy of Sciences of the United States of America 102.39 (2005): 14069-14074.).

The function of the stem cell therapy can be measured by any method known in the art. The function of the stem cell may be measured *in vitro* by any method known in the art including, but not limited to, analysis of gene expression, expression of stem cell markers, cell morphology, cell migration, cell growth, rate and stability of stem cell propagation, spontaneous differentiation, induced differentiation, response to growth factors or expression of lineage markers. Cell-surface antigens and biochemical markers can be assessed by immunocytochemistry, immunofluorescence, fluorescence-activated cell sorting, ELISA, western blotting, qPCR, RNAseq or microarray.

The function of the stem cell may be measured *in vivo.* For example, function of the stem cell can be measured by biopsy of the target tissue and analysis of graft stem cell derived tissue. PCR amplification of variable number tandem repeats (VNTR) loci can be used to identify donor and recipient cells. Further these cells can be characterized in terms of morphology, cell-surface antigens, biochemical markers, gene expression, karyotype analysis, and biologic activity. Cell-surface antigens and biochemical markers can be assessed by immunocytochemistry, immunofluorescence, fluorescence-activated cell sorting, ELISA, western blotting, qPCR, RNAseq or microarray. The efficacy of the therapy can be assayed by measuring the expression of markers for the desired cell type, and the expression of markers for non-desired cell types. The efficacy of the therapy can be assayed by measuring function of the target tissue or cell type. For example, the efficacy of a cardiac stem cell treatment can be investigated through measuring the increase of ejection fraction (EF); the percentage of blood ejected from the left ventricle with each heartbeat, electrocardiogram, echocardiogram, exercise/nuclear stress test, Holter monitoring or by measuring expression of cardiac markers or expression of noncardiac markers, for example, neural markers. In another example, the efficacy of a treatment with neural derived stem cells can be measured by testing for changes in neural functions such as spatial memory, working memory, motor coordination, MRI, PET scans, and quality of life outcomes. In another example, the efficacy of a treatment with hepatocyte stem cells can be measured by a liver enzyme test which are commonly used clinically and measure the presence of liver enzymes such as ALT and AST in the blood. In one aspect of this invention it may improve the migration potential of a stem cell. In some instances, potentiating the function of a stem cell in a subject may refer to improving any aspect of the stem cell therapy. Some non-limiting examples include increasing survival of the stem cell in the subject, increasing the self-renewal of the stem cell in the subject, increasing the replication of transit amplifying cells derived from the stem cell, increasing engraftment of the stem cell into a desired niche in the subject, increasing the migration potential of the stem cell, increasing the homing of the stem cell to a desired tissue or organ, increasing the differentiation of the stem cell into a desired cell type or cell types, decreasing the engraftment of the stem cell into non-desired niches of the subject, decreasing the differentiation of the stem cell towards a non-desired cell type or cell types, decreasing the side effects associated with the treatment, improving the functional recovery obtained from the stem cell treatment, decreasing a required dose of an immunosuppressive drug, or prolonging the life of the stem cell in the subject.

A subject receiving a pharmaceutical composition comprising an encapsulated cannabinoid compound prior to, concurrent with or subsequent to a stem cell therapy may show improved outcomes compared to a subject who does not receive the pharmaceutical composition. For example, a subject who received the pharmaceutical composition may experience fewer side effects, less severe side effects, or a shorter duration of side effects. The subject may show a more rapid recovery, may show a more robust recovery, may maintain a recovered state for longer, or may have improved function of a treated organ or organ system compared to a subject who did not receive the pharmaceutical composition. Examples of side effects which may be reduced include nausea, vomiting, fatigue, temporary hair loss, infection, bleeding, anemia, graft-versus-host disease, veno-occlusive disease, digestive system problems, skin problems, pain, inflammation of the parotid gland, engraftment syndrome, lung problems, kidney problems, nerve and muscle problems, graft failure, graft rejection, heart problems, eye problems, thyroid problems, developmental problems, bladder problems, central nervous system problems, fertility problems and cancer.

The stem cell treatment and cannabinoid composition can be coadministered. The terms "co-administration," "administered in combination with," and their grammatical equivalents, encompass administration of two or more agents to an animal so that both agents and/or their metabolites are present in the animal at the same time. Co-administration includes simultaneous administration in separate compositions, administration at different times in separate compositions, or administration in a composition in which both agents are present. Coadministered agents can be in the same formulation. Co-administered agents may also be in different formulations.

Subjects of the present disclosure can include humans and other animals, such as pets (e.g., dogs, cats, birds, small mammals, snakes) and livestock or farm animals (e.g., cows, pigs, horses, sheep, chickens). Compositions of the present disclosure can be useful for veterinary applications. In some embodiments, the subject of the disclosure described herein is a human.

The subject can have reached adulthood. In some embodiments the subject has not reached adulthood. In some embodiments the subject is a fetus.

The subject can be a human or animal with a disease that is amenable to treatment with stem cells. Examples of diseases that can be treated with stem cells are discussed above. The subject can also be a human or animal with an injury which is amenable to treatment with stem cells. Illustrative examples of injuries that can be treated with stem cells are discussed above.

### Cannabinoid Compositions

A composition administered to a subject to potentiate the function of a stem cell comprises an encapsulated cannabinoid compound. Cannabinoids utilized in the compositions disclosed herein may include but are not limited to cannabigerol-type (CBG), cannabigerolic acid (CBGA), cannabigerolic acid monomethylether (CBGAM), cannabigerol monomethyl ether (CBGM), cannabichromene-type (CBC), cannabichromanon (CBCN), cannabichromenic acid (CBCA), cannabichromevarin-type (CBCV), cannabichromevarinic acid (CBCVA), cannabidiol-type (CBD), tetrahydrocannabinol-type (THC), iso-tetrahydrocannabinol-type (iso-THC), cannabinol-type (CBN), cannabinolic acid (CBNA), cannabinol methylether (CBNM), cannabinol-C₄ (CBN-C₄), cannabinol-C₂ (CBN-C₂), cannabiorcol (CBN-C₁), cannabinodiol (CBND), cannabielsoin-type (CBE), cannabielsoic acid A (CBEA-A), cannabielsoic acid B (CBEA-B), cannabicyclol-type (CBL), cannabicyclolic acid (CBLA), cannabicyclovarin (CBLV), cannabicitran-type (CBT), cannabitriol, cannabitriolvarin (CBTV), ethoxy-cannabitiolvarin (CBTVE), cannabivarin-type (CBV), cannabinodivarin (CBVD), tetrahydrocannabivarin-type (THCV), cannabidivarin-type (CBDV), cannabigerovarin-type (CBGV), cannabigerovarinic acid (CBGVA), cannabifuran (CBF), dehydrocannabifuran (DCBF), and cannabiripsol (CBR) cannabinoids.

Cannabinoids used in compositions and methods of the present disclosure can be derived from various sources, including but not limited to hemp (e.g. hemp stalk, hemp stem, hemp seed), cannabis (e.g., cannabis flower, cannabis leaf, cannabis stalk, cannabis stem, cannabis seed), Echinacea purpurea, Echinacea angustifolia, Echinacea pallida, Acmella oleracea, Helichrysum umbraculigerum, Radula marginata, kava, black truffle, Syzygium aromaticum (cloves), Rosmarinus oficinalis, basil, oregano, black pepper, lavender, true cinnamon, malabathrum, cananga odorata, copaifera spp., and hops.

Encapsulated cannabinoids can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabinoids can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabinoids can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabinoids can be included in a quantity of from about 1 to about 10 micrograms per capsule. Encapsulated cannabinoids can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabinoids can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabinoids can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

Cannabinoids can be formulated in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabinoids can be formulated in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabinoids can be formulated in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabinoids can be formulated in a product, such as a food product, in a quantity of from about 50 to about 150 milligrams. Cannabinoids can be formulated in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%,7 %, 8%, 9%, 10%, 11%, 12%, 13%, 14%, 15%, 16%, 17%, 18%, 19%, 20%, 25%, 30%, 35%, 40%, 45%, or 50% by weight of the product. Cannabinoids can be formulated in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Cannabinoids can be formulated in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The cannabinoids of the encapsulated cannabinoid compounds utilized in a subject method disclosed herein can comprise cannabidiol-class compounds, including but not limited to cannabidiol (CBD), cannabidiolic acid (CBDA), cannabidiol monomethylether (CBDM), cannabidiol-C₄ (CBD-C₄), cannabidivarin (CBDV), cannabidivarinic acid (CBDVA), cannabidiorcol (CBD-C₁), and combinations thereof. CBD can comprise delta-1-cannabidiol, delta-2- cannabidiol, delta-3- cannabidiol, delta-3,7- cannabidiol, delta-4- cannabidiol, delta-5-cannabidiol, delta-6- cannabidiol, and combinations thereof.

Encapsulated cannabidiol compounds can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabidiol compounds can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabidiol compounds can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabidiol compounds can be included in a quantity of from about 1 to about 10 micrograms per capsule. Encapsulated cannabidiol compounds can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabidiol compounds can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabidiol compounds can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

The composition comprising an encapsulated cannabinoid compound may be water soluble. The composition comprising an encapsulated cannabinoid compound may be water soluble even where atmospheric gravity is low relative to sea level. The encapsulated cannabinoid compound may be soluble at temperatures ranging from about 6°C to about 55°C. The encapsulated cannabinoid compound may be soluble at pressures ranging from about 1 atmosphere to about 5 atmospheres. The encapsulated cannabinoid compound may be soluble under ambient conditions. Ambient conditions may be about 15°C to about 30°C and about 1 atmosphere to about 3 atmospheres. For example, about 2.5 milligrams of composition may be soluble in 25 milliliters of water.

Cannabidiol compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabidiol compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabidiol compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabidiol compounds can be included in a product, such as a food product, in a quantity of from about 50 to about 150 milligrams. Cannabidiol compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Cannabidiol compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Cannabidiol compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%. 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The compositions and methods of the present disclosure can comprise tetrahydrocannabinol (THC) as a type of cannabinoids. THC can comprise delta-9-THC, delta-8-THC, and combinations thereof. THC can comprise delta-6a, 7-tetrahydrocannabinol, delta-7-tetrahydrocannabinol, delta-8-tetrahydrocannabinol, delta-9,11- tetrahydrocannabinol, delta-9-tetrahydrocannabinol, delta-10-tetrahydrocannabinol, delta-6a,10a- tetrahydrocannabinol, and combinations thereof. Delta-9- tetrahydrocannabinol can comprise stereoisomers including (6aR,10aR)-delta-9-tetrahydrocannabinol, (6aS, 10aR)-delta-9-tetrahydrocannabinol, (6aS, 10aS)-delta-9-tetrahydrocannabinol, (6aR,10aS)-delta-9-tetrahydrocannabinol, and combinations thereof.

In some embodiments, the capsules may comprise THC. THC compounds can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated THC compounds can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated THC compounds can be included in a quantity of from about 1 to about 10 micrograms per capsule. Encapsulated THC compounds can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated THC compounds can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated THC compounds can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated THC compounds can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

THC compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). THC compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). THC compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). THC compounds can be included in a product, such as a food product, in a quantity of from about 50 to about 150 milligrams. THC compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. THC compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. THC compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

In some cases, a composition of the present disclosure does not contain a psychoactive amount of THC. For example, cannabinoids in compositions of the present disclosure can contain less than 100%, 90%, 80%, 70%, 60%, 50%, 40%, 30%, 20%, 10%, 5%, 1%, 0.7%, 0.5%, 0.3%, or 0.1% THC relative to the total quantity of cannabinoid compounds. In some cases, the ratio of a non-THC cannabinoid (e.g., cannabidiol) to THC in a composition of the present disclosure is greater than or equal to about 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, or 100:1. In some cases, compositions of the present disclosure contain less than 0.3% THC.

In some embodiments, the compositions of the present disclosure may comprise an antioxidant. An antioxidant may inhibit oxidation by reactive species. An antioxidant may refer to a compound that inhibits or delays the oxidation of other molecules by inhibiting the initiation or propagation of oxidizing chain reactions. An antioxidant may be a compound that neutralizes reactive oxygen species that may cause oxidative stress. An antioxidant may be compound that acts as an oxygen scavenger or a chelating agent. An antioxidant may neutralize a free radical by donating one of its own electrons. An antioxidant may be an enzymatic or non-enzymatic molecule. Enzymatic antioxidants include enzymes that metabolize oxidative toxic intermediates, such as reactive oxygen species. Enzymatic molecules include enzymes such as superoxide dismutase, glutathione peroxidase, glutathione reductase, catalases and ascorbate oxidase. Non-enzymatic molecules may include glutathione, vitamin C, uric acid, albumin, bilirubin, vitamin E (α- tocopherol), carotenoids (e.g., β-carotene), and flavonoids.

The compositions of the present disclosure can comprise one or more terpene compounds, including but not limited to terpenoids such as monoterpenoids, sesquiterpenoids, diterpenoids, and triterpenoids. Terpenes can be acyclic, monocyclic, or polycyclic. Terpenes can include but are not limited to myrcene, limonene, linalool, trans-ocimene, cis-ocimene, alpha-pinene, beta-pinene, alpha-humulene (alpha-caryophyllene), beta-caryophyllene, delta-3-carene, trans-gamma-bisabolene, cis-gamma-bisabolene, trans-alpha-farnesene, *cis*-beta-farnesene, beta-fenchol, beta-phellandrene, guajol, alpha-gualene, alpha-eudesmol, beta-eudesmol, gamma-eudesmol, terpinolene, alpha-selinene, beta-selinene, alpha-terpineol, fenchone, camphene, cis-sabinene hydrate, alpha-trans-bergamotene, alpha-cis-bergamotene, borneol, gamma-curcumene, alpha-thujene, epi-alpha-bisabolol, ipsdienol, alpha-ylangene, beta-elemene, gamma-muurolene, alpha-cadinene, alpha-longipinene, caryophyllene oxide, and combinations thereof.

Encapsulated terpenes can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated terpenes can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated terpenes can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated terpene compounds can be included in a quantity of from about 1 to about 10 micrograms per capsule. Encapsulated terpenes can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated terpenes can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated terpenes can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

Terpene compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Terpene compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Terpene compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Terpene compounds can be included in a product, such as a food product, in a quantity of from about 50 to about 150 milligrams. Terpene compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Terpene compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Terpene compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The compositions of the present disclosure can be enriched in cannabinoids compared to hemp oil. For example, a composition can comprise hemp oil and cannabinoids from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of cannabinoids compared to hemp oil.

The compositions of the present disclosure can be enriched in cannabidiol compounds compared to hemp oil. For example, a composition can comprise hemp oil and cannabidiol compounds from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of cannabidiol compounds compared to hemp oil.

The compositions of the present disclosure can be enriched in THC compounds compared to hemp oil. For example, a composition can comprise hemp oil and THC compounds from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of THC compounds compared to hemp oil.

The compositions of the present disclosure can be enriched in terpenes compared to hemp oil. For example, a composition can comprise hemp oil and terpenes from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of terpenes compared to hemp oil.

Encapsulated cannabinoid compounds included in the compositions of the present disclosure can be derived from various sources. Compound sources can be natural, such as plant extracts or essential oils. Compounds in the compositions of the present disclosure can be derived from hemp oil, including cannabinoid compounds, THC compounds, and terpene compounds. Compounds in the compositions of the present disclosure can be derived from essential oils, including but not limited to those essential oils discussed further in this disclosure. These compounds can include cannabinoid compounds and terpene compounds. In some cases, all the compounds or ingredients in a composition are natural or naturally-derived. In some cases, all the compounds or ingredients in a composition are vegetarian. In some cases, all the compounds or ingredients in a composition are vegan.

Terpenes and/or essential oils in compositions of the present disclosure can be selected to provide benefits for particular conditions or subjects. Terpenes and/or essential oils can be employed in combination with each other, as well as in combination with cannabinoids, for example, reduce cellular oxidative stress, and, in some instances, ameliorate one or more health conditions. For example, terpinolene, terpineol and linalool or lavender, valerian and jasmine essential oils can be combined with cannabinoids or cannabis extract to act as a sleep aid or treat sleep disorders.

Alpha-pinene can be used as an anti-inflammatory, an antiangiogenic, an anti-ulcer agent, and a bronchodilator.

Linalool can be used for reducing anxiety, reducing inflammation (e.g., lung inflammation), to improve Alzheimer's disease or symptoms thereof, as a sedative, an analgesic, an anti-microbial, an antibacterial, and an anti-epileptic.

Myrcene can be used as an antibacterial, a neuroprotective agent, an antinociceptive, an analgesic, and to alleviate neuropathic pain, peptic ulcer disease, and inflammation. Depending on concentration, myrcene can be used as a sedative (e.g., over 0.5% myrcene) or to provide energizing effects (e.g., less than 0.5% myrcene).

Limonene can be used to reduce anxiety and depression, to dissolve cholesterol-containing gallstones, to neutralize gastric acid, support normal peristalsis, relieve heartburn and gastroesophageal reflux, to improve immune function, and as a chemopreventative against cancer.

Ocimene can be used as an antifungal agent, an antitumor agent, and a cyctotoxic agent.

Terpinolene can be used for antioxidant, mood regulation, central nervous system (CNS) regulation, anti-inflammatory, anti-diarrheal, anti-filarial, anti-fungal, antimalarial, anti-amoebic, anti-bacterial, cytotoxic, and anticancer effects.

Terpineol can be used to relax a subject, to aid digestion and improve gastrointestinal disorders, and to relieve influenza, bronchitis, cough, nasal congestion, and sinusitis.

Beta-caryophyllene can be used as an anti-inflammatory agent, an anti-tumor agent, and an analgesic.

Geraniol can be used to reduce or protect against neuropathy, as an antidepressant, to suppress angiogenesis, to improve anti-cancer agent efficacy, to suppress growth of cancer cells (e.g., lung cancer), as a chemopreventive against cancer, to reduce inflammation and apoptosis (e.g., in liver cells), to reduce oxidative stress, as an antioxidant, and as an antimicrobial.

Alpha-humulene can be used as an appetite suppressant, an anti-inflammatory agent, an insect repellant, an antibacterial, an antioxidant, and an allelopathic agent.

Phellandrene can be used as an antidepressant and an antihyperalgesic.

Carene can be used as an antioxidant, an antiproliferative, an antimicrobial, and to reduce excess body fluid production, such as of tears, mucous, or sweat.

Terpinene can be used as an antioxidant, an anti-inflammatory, an antimicrobial, an antiproliferative, to reduce oxidative stress, and to manage diabetes.

Fenchol can be used as an antibacterial agent, an antimycobacterial, an antimicrobial, and an antioxidant.

Borneol can be used to alleviate hyperalgesia, as a TRPA1 inhibitor, an anti-inflammatory agent, and an anti-nociceptive agent.

Bisabolol can be used as an anti-cancer agent, such as to induce apoptosis in leukemia, an anti-tumor agent (e.g., pancreatic cancer), and an antigenotoxicity agent.

Phytol can be used to relax a subject, such as by inhibiting degradation of GABA, as an anxiolytic, to resist menadione-induced oxidative stress, and as an antimicrobial.

Camphene can be used for pain relief, as an antioxidant, to induce apoptosis in cancer cells (e.g., melanoma), an antitumor agent, and an antibacterial.

Sabinene can be used as an antioxidant, an antimicrobial, an anticancer agent (e.g., oral, liver, lung, colon, melanoma, and leukemic cancer), to aid liver function, aid digestion, relieve arthritis, and relieve skin conditions.

Camphor can be used to improve skin healing (e.g., reconstructed human epidermis), as a local anesthetic, a muscle relaxant, an antipathogenic, and an antimicrobial agent.

Isoborneol can be used as an antioxidant, a cytotoxic, a DNA-protective, to inhibit herpes simplex virus type 1, and to inhibit HIV.

Menthol can be used as an analgesic, to desensitize α3β4 nicotinic acetylcholine receptors, as an antinociceptive, and as an anti-inflammatory agent.

Nerolidol can be used as an antifungal agent, an antimicrobial agent, an antioxidant, and an antimalarial agent.

Guaiol can be used as an antimicrobial agent, an antifungal agent, and an antibiotic.

Isopulegol can be used as a gastroprotective agent, an anti-inflammatory agent, to enhance permeability for transdermal administration of compounds, and to reduce the severity of seizures.

Geranyl acetate can be used as an antimicrobial agent, an antibacterial, and an antioxidant.

Cymene can be used as an anti-inflammatory agent, an anti-hyperalgesic, an antioxidant, an anti-diabetic, to aid in weight loss, to aid immune disorders, and to protect against acute lung injury.

Eucalyptol can be used as an antifungal agent, to alleviate inflammation (e.g., lung inflammation), an antioxidant, and an anticancer agent.

Pulegone can be used to enhance skin permeability, as an insecticide, and an antioxidant.

The compositions of the present disclosure can comprise one or more essential oils or essential oil compounds. Essential oils can include, but are not limited to: Linalool; B-Caryophyllene; B-Myrcene; D-Limonene; Humulene; a-Pinene; Ylang Ylang (Cananga odorata); Yarrow (Achillea millefolium); Violet (Viola odorata); Vetiver (Vetiveria zizanoides); Vanilla (Vanilla plantifolia); Tuberose (Polianthes tuberosa); Thyme (Thymus vulgaris L.); Tea Tree (Melaleuca alternifolia); Tangerine (Citrus reticulata); Spruce, Black (Picea mariana); Spruce (Tsuga Canadensis); Spikenard (Nardostachys jatamansi); Spearmint (Mentha spicata); Sandalwood (Santalum spicatum); Rosewood (Aniba rosaeodora); Rosemary Verbenone (Rosmarinus officinalis); Rosemary (Rosmarinus officinalis); Rose (Rosa damascena); Rose Geranium (Pelargonium roseum); Ravensara (Ravensara aromatica); Plai (Zingiber cassumunar) Pine Needle (Pinus sylvestris L.); Petitgrain (Citrus aurantium); Peppermint (Mentha piperita); Pepper, Black (Piper nigrum L.); Patchouli (Pogostemon cablin); Palo Santo (Bursera graveolens); Palmarosa (Cymbopogon martini); Osmanthus (Osmanthus fragrans); Oregano (Origanum vulgare); Orange, Sweet (Citrus sinensis); Oak Moss (Evernia prunastri); Nutmeg (Myristica fragrans) Niaouli (Melaleuca viridifloria); Neroli (aka Orange Blossom) (Citrus aurantium); Myrtle (Myrtus communis); Myrrh (Commiphora myrrha); Mimosa (Acacia decurrens); Melissa (Melissa officinalis L.); Marjoram, Sweet (Origanum majorana); Manuka (Leptospermum scoparium); Mandarin, Red (Citrus deliciosa); Mandarin (Citrus deliciosa); Lotus, White (Nelumbo nucifera); Lotus, Pink (Nelumbo nucifera); Lotus, Blue (Nelumbo nucifera); Lime (Citrus aurantifolia); Lily (Lilum aurantum); Lemongrass (Cymbopogon citratus); Lemon (Citrus limonum); Lavender (Lavandula angustifolium); Lavandin (Lavandula hybrida grosso); Kanuka (Kunzea ericoides); Juniper Berry (Juniperus cummunis); Jasmine (Jasminum officinale); Jasmine Abs (Jasminum sambac); Helichrysum (Helichrysum italicum); Grapefruit, White (Citrus x paradisi); Grapefruit, Pink (Citrus paradisi); Ginger (Zingiber officinalis); Geranium (Pelargonium graveolens); Geranium, Bourbon (Pelargonium graveolens, 'Herit); Gardenia (Gardenia jasminoides); Galbanum (Ferula galbaniflua); Frankincense (Boswellia carterii); Frangipani (Plumeria alba); Fir Needle White (Abies alba); Fir Needle Siberia (Abies siberica); Fir Needle Canada (Abies balsamea); Fennel, Sweet (Foeniculum vulgare); Eucalyptus Smithii. Eucalyptus Radiata, Eucalyptus Globulus, Eucalyptus Citriodora, Eucalyptus Blue Mallee (Eucalyptus polybractea); Elemi (Canarium luzonicum); Dill (Anethum graveolens); Cypress (Cupressus sempervirens); Cumin (Cuminum cyminum); Coriander (Coriandum sativum); Cocoa (Theobroma cacao); Clove (Eugenia caryophylatta); Clary Sage (Salvia sclarea); Cistus (aka Labdanum) ( Cistus ladaniferus L.); Cinnamon (Cinnamomum zeylanicum); Chamomile, Roman (Anthemis nobilis); Chamomile, Blue (Matricaria chamomilla); Celery Seed (Apium graveolins); Cedarwood, Western Red (Thuja plicata); Cedarwood, Blood (Juniperus virginiana); Cedarwood Atlas (Cedrus atlantica); Carrot Seed (Daucus carota); Cardamon (Elettaria cardamomum); Caraway Seed (Carum carvi); Cajeput (Melaleuca cajuputi); Cade (Juniperus oxycedrus); Birch, White (Betula alba); Birch, Sweet (Betula lenta); Bergamot (Citrus bergamia); Bay Laurel (Laurus nobilis); Basil (Ocimum basilicum); Basil, Holy (Ocimum sanctum); Basil (Ocimum basilicum); Balsam Poplar (Populus balsamifera); Balsam Peru (Myroxylon balsamum); Angelica (Angelica archangelica L.); and combinations thereof.

The compositions of the present disclosure can comprise one or more additional ingredients, including but not limited to mushrooms or mushroom derivative products (e.g., reishi mushroom, chaga mushroom, maitake mushroom, oyster mushroom, cordyceps), maca (Lepidium meyenii), he sho wu (also he show wu or shou wu chih), superfoods or superfood derivative products (e.g., blueberries, acai berries, inca berries, goji berries, camucamu, coconut, lucuma, kale, cacao (e.g., cacao powder, cacao butter), sacha inchi, chia, flax, hemp, amaranth, quinoa, moringa oleifera), and combinations thereof.

Compounds used in compositions of the present disclosure can be extracted by a variety of methods. For example, extraction can be performed by maceration, infusion, decoction, percolation, Soxhlet extraction, pressurized solvent extraction, counter current extraction, ultrasonication, or supercritical fluid (e.g., carbon dioxide) extraction.

In some cases, compounds used in compositions of the present disclosure are extracted via supercritical fluid (e.g., carbon dioxide) extraction. For example, cannabinoid compounds can be extracted from hemp (e.g., hemp stalk and hemp stems) using supercritical carbon dioxide extraction.

The compositions of the present disclosure can comprise pregnenolone, including derivatives thereof. Pregnenolone can help protect a subject from cannabis intoxication, for example, from THC. Pregnenolone or derivatives thereof can be formulated to be water soluble. A composition of the present disclosure can comprise between about 1 and 50 milligrams (mg) of pregnenolone or derivatives thereof. For example, a unit dosage of the present disclosure can comprise between about 1 and 50 milligrams (mg) of pregnenolone. Compositions of the present disclosure (e.g., unit dosages) can comprise about 1, 2, 3, 4, 5, 6, 7, 8, 9, 10, 15, 20, 25, 30, 35, 40, 45, or 50 mg of pregnenolone. Compositions of the present disclosure (e.g., unit dosages) can comprise at least about 1, 2, 3, 4, 5, 6, 7, 8, 9, 10, 15, 20, 25, 30, 35, 40, 45, or 50 mg of pregnenolone. Compositions of the present disclosure (e.g., unit dosages) can comprise at most about 1, 2, 3, 4, 5, 6, 7, 8, 9, 10, 15, 20, 25, 30, 35, 40, 45, or 50 mg of pregnenolone. Compositions comprising pregnenolone can be used in combination with any other compounds, ingredients, or formulations described herein, including esters, cyclodextrin complexes, capsules (e.g., sodium alginate capsules), immediate release formulations, delayed or extended release formulations, transbuccal formulations, and sublingual formulations.

The compositions of the present disclosure comprise encapsulated cannabinoid compounds. Capsules can comprise components discussed in this disclosure, such as cannabinoid compounds. In some embodiments, compositions comprising terpene compounds, cannabidiol, THC, and/or others, in may also be encapsulated. In some cases, compositions can be encapsulated without the use of liposomes. In some cases, compositions can be encapsulated without the use of micelles. In some cases, compositions can be encapsulated without the use of liposomes or micelles. Compounds of the composition can exist be encapsulated in forms including but not limited to liquid, gel, semi-solid, and solid. Encapsulated compositions disclosed herein can further be processed into forms including but not limited to solids, powders, liquids, suspensions, gels, tablets, foods, lotions, cosmetics, and other forms discussed in this disclosure.

Encapsulation can be performed with a encapsulation device, including microfluidic droplet generation or encapsulation devices. An exemplary microencapsulation device is described, for example, in U.S. Patent No. 7,482,152, which is entirely incorporated herein by reference. Microfluidic droplets or emulsions can be generated by flow of a fluid to be encapsulated with an immiscible carrier fluid. For example, an oil fluid to be encapsulated can be flowed with an aqueous carrier fluid, or an aqueous fluid to be encapsulated can be flowed with an oil carrier fluid. Air can also be used as a fluid. Microfluidic droplet generators useful for microencapsulation include those employing co-flowing streams, cross-flowing streams (e.g., flow of streams at a T-junction), flow focusing, flow through perforated plates, and flow through nozzles. Droplet size can be controlled by parameters including device geometry, relative flow rates of the fluid streams, and operating pressure.

Examples of encapsulated compositions, including encapsulated cannabinoid compositions, are provided in WO/2016/094810, which is entirely incorporated herein by reference.

Encapsulation can be performed at a range of operating parameters, such as different flow rates or pressures. Encapsulation can be conducted at a pressure of at least about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, 50000 psi, or more. Encapsulation can be conducted at a pressure of at most about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, or 50000 psi. Microencapsulation can be conducted at a pressure of about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, 50000 psi, or more. Encapsulation can be conducted at a flow rate of at least about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, 500 mL/min, or more. Encapsulation can be conducted at a flow rate of at most about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, or 500 mL/min. Encapsulation can be conducted at a flow rate of about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, 500 mL/min, or more.

Droplet generators can employ multiple parallel droplet generation operations in parallel. For example, a droplet generator (e.g., a plate, a device with channels) can employ at least 1, 2, 3, 4, 5, 6, 7, 8, 9, 10, 15, 20, 25, 30, 35, 40, 45, 50, 55, 60, 65, 70, 75, 80, 85, 90, 95, 100, or more droplet generating features (e.g., holes, channels, nozzles). A droplet generator can employ at most 1, 2, 3, 4, 5, 6, 7, 8, 9, 10, 15, 20, 25, 30, 35, 40, 45, 50, 55, 60, 65, 70, 75, 80, 85, 90, 95, or 100 droplet generating features. A droplet generator can employ about 1, 2, 3, 4, 5, 6, 7, 8, 9, 10, 15, 20, 25, 30, 35, 40, 45, 50, 55, 60, 65, 70, 75, 80, 85, 90, 95, 100, or more droplet generating features.

Encapsulation can be performed via an emulsification process. For example, compositions can be emulsified in a mixer, such as an agitator, impeller, centrifugal mixer, or high-shear mixer. High-shear mixers can include batch high-shear mixers and inline high-shear mixers (e.g., rotor-stator mixers). Emulsification can also be conducted without a mixer, by combining fluids thermodynamically favored to form an emulsion, optionally with the aid of one or more emulsifiers or surfactants.

Encapsulation processes can be conducted with the aid of one or more emulsifiers or surfactants. Emulsifiers and surfactants can include but are not limited to saponins (e.g., quillaja tree extract such as Q-NATURALE^{®}, yucca extract), lecithin, soy lecithin, mustard seed hull extract, sodium stearoyl lactylate, polysorbate 20, and combinations thereof.

Encapsulated cannabinoid compounds can comprise one or more stabilizers or gelling agents, which can be used to stabilize a capsule or emulsion. Stabilizers or gelling agents can include but are not limited to alginate (also algin or alginic acid) and agar. Alginate can be used in a variety of forms, including but not limited to inorganic salts such as sodium alginate, potassium alginate, calcium alginate, and combinations thereof. Alginate can be derived from sources such as seaweed (e.g., *Macrocystis pyrifera, Ascophyllum nodosum, Laminaria* spp.) or bacteria (e.g., *Pseudomonas* spp., *Azotobacter* spp.). Cross-linking agents or solutions, such as calcium chloride, can be used to stabilize or gel capsules.

Encapsulated cannabinoid compounds can be characterized by a size (e.g., a diameter). The capsule (e.g., droplet) size can be about 0.154 micrometers. The capsule size can be less than or equal to about 0.154 micrometers. The capsule size can be greater than or equal to about 0.154 micrometers. The capsule size can be about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 150, 200, 250, 300, 350, 400, 450, or 500 micrometers. The capsule size can be less than or equal to about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 150, 200, 250, 300, 350, 400, 450, or 500 micrometers. The capsule size can be greater than or equal to about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 150, 200, 250, 300, 350, 400, 450, or 500 micrometers. The capsule size can be from about 0.1 to about 0.2 micrometers. The capsule size can be from about 0.05 to about 0.25 micrometers. The capsule size can be from about 0.05 to about 0.55 micrometers. The capsule size can be from about 0.05 to about 1 micrometers. The size distribution in a population of capsules can be homogeneous or substantially homogeneous. For example, a population of capsules can be characterized by dispersity, or polydispersity index (PDI), of less than or equal to about 20, 19, 18, 17, 16, 16, 15, 14, 13, 12, 11, 10, 9, 8, 7, 6, 5, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.45, 1.40, 1.35, 1.30, 1.25, 1.20, 1.15, 1.14, 1.13, 1.12, 1.11, 1.10, 1.09, 1.08, 1.07, 1.06, 1.05, 1.04, 1.03, 1.02, 1.01, or 1.00.

An effective amount of a composition comprising an encapsulated cannabinoid compound is administered to a subject. The term "effective amount" or "therapeutically effective amount" refers to that amount of a encapsulated cannabinoid compound described herein that is sufficient to effect the intended application including but not limited to a potentiation of function of a stem cell and/or disease treatment in a subject. The therapeutically effective amount may vary depending upon) the subject and condition being treated, e.g., the weight and age of the subject, the severity of the disease condition, the manner of administration and the like, which can readily be determined by one of ordinary skill in the art. The term also applies to a dose that will induce a particular response in target cells, e.g., a change in expression of a stem cell marker. The specific dose will vary depending on the particular formulation of the encapsulated cannabinoid compound, the dosing regimen to be followed, whether it is administered in combination with other compounds, timing of administration, the tissue to which it is administered, route of administration and the physical delivery system in which it is carried.

Any of the subject compositions can be provided in a unit dosage form. A unit dosage is an amount of a compound, such as a cannabinoid compound delivered alone or in combination with other components, which is to be administered to a subject at or about one time point. Other components which can be included with a unit dosage include but are not limited to cosmetics, food carriers, food bars, baked goods, dairy products, oils, beverages, solid dosages (e.g., tablets), or liquid dosages. A unit dosage of a cannabinoid compound can be about 10, 20, 30, 40, 50, 60, 70, 80, 90, 100, 125, 150, 175, 200, 250, 300, 350, 400, 450, 500, 600, 700, 800, 900, 1000, 1100, 1200, 1300, 1400, 1500, 1600, 1700, 1800, 1900, 2000 or more milligrams (mg). A unit dosage of a cannabinoid compound can be at least about 10, 20, 30, 40, 50, 60, 70, 80, 90, 100, 125, 150, 175, 200, 250, 300, 350, 400, 450, 500, 600, 700, 800, 900, 1000, 1100, 1200, 1300, 1400, 1500, 1600, 1700, 1800, 1900, 2000 or more milligrams (mg). A unit dosage of a cannabinoid compound can be at most about 10, 20, 30, 40, 50, 60, 70, 80, 90, 100, 125, 150, 175, 200, 250, 300, 350, 400, 450, 500, 600, 700, 800, 900, 1000, 1100, 1200, 1300, 1400, 1500, 1600, 1700, 1800, 1900, 2000 or more milligrams (mg). A unit dosage can be an hourly dosage. A unit dosage can be a daily dosage. A unit dosage can provide about 1/24, 1/12, 1/8, 1/6, 1/4, 1/3, 1/2, or all of a daily dosage of one or more cannabinoids for a subject. A unit dosage can take the form of a tablet, gel, liquid, food product, food bar, container of liquid of defined volume, or other forms described herein, packaged for one-time consumption or administration.

The amount of the composition of the subject method administered may be dependent on the subject being treated, the severity of disorder or condition, the rate of administration, the disposition of the composition and encapsulated cannabinoid compound. The amount of the composition administered may also depend on the type and amount of stem cells administered. An effective dosage is in the range of about 0.1 mg to about 2000 mg per kg body weight per day. For example, for a 70 kg human, this may amount to about 7 mg/day to about 1.75 g/day. In some instances, dosage levels below the lower limit of the aforesaid range may be more than adequate, while in other case still larger doses may be employed without causing any harmful side effects, e.g. by dividing such larger doses in several small doses for administration throughout the day. Dosages may be administered over time periods of hours, days, weeks, or months.
The composition comprising an encapsulated cannabinoid compound can be administered to a subject in any of several ways to potentiate the function of a stem cell. The compositions can be administered, for example, orally or transdermally. Other delivery routes include but are not limited to intranasal, sublingual, transmucosal, or intradermal. An effective amount of the compositions may be in in dosage unit formulations containing conventional nontoxic physiologically acceptable carriers, adjuvants, and vehicles as desired.

Oral administration may be accomplished using an oral dosage form. Oral dosage forms can be solid (e.g., a tablet or bulk powder) or liquid (e.g., a suspension or slurry). Tablets can include tablets, caplets, capsules, including soft gelatin capsules, and lozenges. Tablets can further comprise suitable binders, lubricants, diluents, disintegrating agents, colorants, flavoring agents, flow-inducing agents, and melting agents.

Compositions comprising an encapsulated cannabinoid compound formulated for oral administration can be incorporated into a food composition. A food composition can be a beverage, a solid food, or a semi-solid food. Food compositions can comprise an encapsulated cannabinoid compound and a food carrier. A food carrier can be practically any food product. Examples of food carriers include, but are not limited to food bars (granola bars, protein bars, candy bars, etc.), cereal products (oatmeal, breakfast cereals, granola, etc.), bakery products (bread, donuts, crackers, bagels, pastries, cakes, etc.), beverages (milk-based beverage, sports drinks, fruit juices, alcoholic beverages, bottled waters), pastas, grains (rice, corn, oats, rye, wheat, flour, etc.), egg products, snacks (candy, chips, gum, chocolate, etc.), meats, fruits, and vegetables.

A composition comprising an encapsulated cannabinoid compound can be administered transdermally, such as via a patch. The compositions of the present disclosure can be administered intravenously. The compositions of the present disclosure can be administered topically. The compositions of the present disclosure can be administered via topical exposure to an aqueous solution, such as immersing a subject in a float tank. The compositions of the present disclosure can be formulated as a bath salt or liquid bath product, which can be dissolved or dispersed in water (e.g., a bath) for skin exposure, for example, by immersion of the subject. Compositions comprising an encapsulated cannabinoid compound for topical or transdermal application can be provided as cosmetics or personal care products, such as soaps (e.g., solid, bar, liquid, or foaming), hand sanitizer, lotions, massage oils masks, makeup, moisturizers, sunscreen, toothpaste, mouth wash, or throat spray.

To potentiate the function of a stem cell, a composition comprising an encapsulated cannabinoid compound can be administered using a variety of different mechanisms. The composition may be nebulized, delivered in an aerosolized an aerosol spray preparation from a pressurized pack, or from a dry powder inhaler. Suitable propellants that can be used in a nebulizer include, for example, dichlorodifluoro-methane, trichlorofluoromethane, dichlorotetrafluoroethane and carbon dioxide. The dosage can be determined by providing a valve to deliver a regulated amount of the compound in the case of a pressurized aerosol.

The composition can be administered in a variety of forms to a subject to potentiate the function of a stem cell. The composition can be administered as a dry powder. For example, an oil-based composition (e.g., hemp oil) can be combined with a drying or powdering agent, such as cyclodextrin. In some cases, a powder composition can be provided on its own. In other cases, a powder composition can be provided in another product or composition, such as a food product or composition, cosmetic product, or other products and compositions such as those disclosed herein.

A composition comprising an encapsulated cannabinoid compound can be administered in any suitable form, including but not limited to a liquid form, a gel form, a semiliquid (e.g., a liquid, such as a viscous liquid, containing some solid) form, a semi-solid (a solid containing some liquid) form, or a solid form. Compositions can be provided in, for example, a tablet form, a capsule form, a food form a chewable form, a non-chewable form, a transbuccal form, a sublingual form, a slow-release form, a non-slow-release form, a sustained release form, or a non-sustained-release form.

Compositions comprising an encapsulated cannabinoid compound for inhalation or insufflation include solutions and suspensions in pharmaceutically acceptable, aqueous or organic solvents, or mixtures thereof, and powders. The liquid or solid compositions may contain suitable excipients as set out above. Preferably the compositions of the present invention are administered by the oral, intranasal or respiratory route for local or systemic effect. Compositions in acceptable solvents may be nebulized by use of inert gases. Nebulized solutions may be inhaled directly from a nebulizing device or a nebulizing device may be attached to a face mask, tent or intermittent positive pressure breathing machine. Solution, suspension or powder compositions may be administered orally or nasally from devices that deliver the formulation in an appropriate manner.

The compositions of the present disclosure can comprise an additional agent or agents, whether active or passive. Examples of such an agent include a sweetening agent, a flavoring agent, a coloring agent, a filling agent, a binding agent, a lubricating agent, an excipient, a preservative, or a manufacturing agent. Additional pharmaceutically acceptable excipients (in the case of pharmaceuticals) or other additives (for non-pharmaceutical applications) can be added to the composition. For example, if desired, any generally accepted soluble or insoluble inert pharmaceutical filler (diluent) material can be included in the final product (e.g., a solid dosage form). Such inert pharmaceutical filler can comprise a monosaccharide, a disaccharide, a polyhydric alcohol, inorganic phosphates, sulfates or carbonates, and combinations thereof. Examples of suitable inert pharmaceutical fillers include sucrose, dextrose, lactose, xylitol, fructose, sorbitol, calcium phosphate, calcium sulfate, calcium carbonate, microcrystalline cellulose, and combinations thereof. An effective amount of any generally accepted pharmaceutical lubricant, such as calcium or magnesium soaps, can be added.

In a related but separate aspect, the present disclosure provides a food composition comprising an effective amount of an encapsulated cannabinoid for potentiating the function of a stem cell and a food carrier. Any type, amount, or form of composition comprising an encapsulated cannabinoid disclosed herein is applicable for preparing the food composition disclosed herein.

A food composition or food product can comprise a food bar, including but not limited to granola bars, protein bars, candy bars, and energy bars. A food composition or food product can comprise a cereal product, including but not limited to oatmeal, flour (e.g., wheat flour, rice flour, corn flour, barley flour), breakfast cereal, granola, bread, pasta, rice cakes, and popcorn. A food composition or food product can comprise a bakery product, including but not limited to bread, pastries, brownies, cakes, pies, donuts, crackers, and muffins. A food composition or food product can comprise a dairy product, including but not limited to milk, fermented milk, curd, whey, yogurt, cream, cheese, butter, clarified butter, ghee, and ice cream. A food composition or food product can comprise a nut butter or seed butter, including but not limited to peanut butter, almond butter, cashew butter, hazelnut butter, macadamia nut butter, pecan butter, pistachio butter, walnut butter, pumpkin seed butter, sesame seed butter, soybean butter, and sunflower seed butter. A food composition or food product can comprise an oil (e.g., a cooking oil), including but not limited to olive oil, coconut oil, vegetable oil, canola oil, corn oil, peanut oil, sunflower seed oil, almond oil, avocado oil, rice bran oil, cottonseed oil, flaxseed oil, linseed oil, grape seed oil, hemp oil, mustard oil, macadamia oil, palm oil, tea seed oil, walnut oil, margarine, lard, butter, clarified butter, ghee, or tallow. A food composition or food product can comprise sports food products such as energy gels, sports drinks, energy powders, energy bars, energy shots, protein powders, and protein drinks (e.g., protein shakes). A food composition or food product can comprise a beverage, including but not limited to water, electrolyte drinks, soda, coconut water, tea (e.g., Jun tea, black tea, green tea, white tea, herbal tea), coffee, a soft drink, an alcoholic beverage (e.g., cocktail, liquor, spirits, beer, wine, malt beverage), water, juice (e.g., apple juice, orange juice, tomato juice, vegetable juice, cranberry juice), a sports drink, electrolyte-enriched water, vitamin-enhanced water, a hangover-recovery drink, milk (e.g., dairy-based milk, coconut milk, almond milk, soy milk, hemp milk, rice milk, oat milk, cashew milk, hazelnut milk), and yogurt. A food composition or food product can comprise a fungus or fermented food or drink, including but not limited to kifir (kefir), jun, amasi, amazake, appam, ayran, doogh, bagoong, brem, cheonggukjang, chicha, kombucha, fermented bean curd, kimchi, lassi, miso, poi, yakult, and yogurt.

Compositions of the present disclosure can comprise pet or other animal products, such as animal food (e.g., dog food, cat food), treats, and nutritional supplements (e.g., liquids, sprays, or powders for application to food or water). These compositions can be formulated for or administered to domestic or pet animals (e.g., dogs, cats, small mammals, birds), livestock and other farm animals (e.g., cows, pigs, horses, sheep, goats), zoo animals, or any other vertebrates. Compositions for administration to animals can be formulated with encapsulated cannabinoid-rich oil or non-encapsulated cannabinoid-rich oil, alone or in combination with essential oils, terpenes, and other components described herein. Compositions for administration to animals can be mixed into feed or water, prepared for spraying application (e.g., mixed in glycerin), for intravenous administration (e.g., in a syringe or an IV bag), in salves, vitamins, liquid vitamin pumps, treats, or other forms.

Packaging of a food composition comprising the encapsulated cannabinoid may take the form of several conformations. Packaging may be configured for single service. Packaging may be disposable. As non-limiting examples, packaging may be a pouch, a can, a flexible container, or a sealed tube. Food packaging may be made of any of a variety of materials including Mylar^{®}, Aclar^{®}, polyethylene, foil, and aluminum. Food compositions may be packaged in a retort pouch or a flexible bowl with a lid, food condiment pouches, or full panel pull out cans. Food composition packaging may incorporate utensils such as forks, spoons, knives, or scissors. Packaging may be configured for partial removal to facilitate consumption. For example, a package may remain sealed during rehydration or reheating; for consumption, a portion of the package may be removed. Packaging may be configured for the rehydration of dehydrated food composition powders. For instance, food and beverage packaging may additionally comprise a septum adapter to enable dehydrated food to be rehydrated. Sealed containers comprising food compositions may be configured to be secured in a tray. Dry, powdered, or foods subject to crumbling may be coated in gelatin to reduce crumbling. Oxygen may be removed from a package and replaced with a nitrogen gas and an oxygen scavenger. Packages may be flushed with nitrogen gas before sealing. Packages may be sealed with a vacuum seal. The package may be sealed at about 15 to about 35 inches of Hg vacuum.

A food composition comprising an effective amount of an encapsulated cannabinoid compound may be included in a kit. A kit may be configured for consumption by a subject under a variety of conditions.

In addition to potentiating the function of a stem cell, administering the compositions of the present disclosure to a subject can provide one or more additional beneficial effects. Beneficial effects can include but are not limited to pain relief, reduced bacterial growth, reduced blood sugar levels, improved blood lipid and cholesterol profiles, increased fat burning, reduced appetite, stimulated appetite, reduced vomiting or nausea, reduced seizures or convulsions, antifungal effects, reduced inflammation, reduced arthritis (e.g., rheumatoid arthritis), reduced insomnia or aided sleep, reduced arterial blockage, inhibited cancer cell growth, improved psoriasis, tranquilizing effects, antispasmodic effects, reduced anxiety, bone growth promotion, reduced intestinal contractions, and nervous system protection.

The compositions described herein can provide several advantages, including but not limited to increased shelf stability, increased bioavailability, increased bioactivity, and delayed release. The compositions described herein, when administered to a subject, can have various release profiles, half-lives, and metabolic characteristics. The subject compositions can comprise a plurality of capsules, wherein an individual capsule in the plurality is characterized by exhibiting at least one of: (a) a sigmoidal release profile of the at least one cannabinoid compound; (b) a plasma half-life of the at least one cannabinoid compound greater than twice that of the at least one cannabinoid compound in non-encapsulated form; (c) a first pass metabolism of the at least one cannabinoid compound reduced by at least 50% compared to the at least one cannabinoid compound in non-encapsulated form; d) a rate of excretion of the at least one cannabinoid compound from a subject's body reduced by at least 20% compared to the at least one cannabinoid compound in non-encapsulated form; or (e) a degradation rate at an ambient temperature of at least 20 °C of the at least one cannabinoid compound of less than about 50% of a degradation rate of the at least one cannabinoid compound in non-encapsulated form.

The compositions described herein can have a shelf half-life of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 240, 270, 300, 330, or 360 days. In some cases, the compositions described herein can have a shelf half-life of at least about 1, 2, 3, 4, or 5 years. Compositions in encapsulated form can be characterized by a cannabinoid degradation rate at an ambient temperature of at least 20 °C of at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than the degradation rate of a non-encapsulated cannabinoid composition.

Cannabinoid compositions in encapsulated form can be characterized by a plasma half-life in a subject of at least 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, or 5.0 times that of a non-encapsulated cannabinoid composition. Plasma half-life of a composition can be determined experimentally by administering the composition to a subject, taking plasma samples from a subject at multiple time points, and measuring the concentration of the compound or compounds of interest in those plasma samples. The concentration of the compound or compounds of interest will reach a peak value in the plasma, then fall as the compound or compounds are metabolized, degraded, or cleared from the blood stream. The plasma half-life is the time for the plasma concentration value to be halved.

The cannabinoid release profile can be sigmoidal (e.g., having an 'S' shape curve, such as a logistic function). The cannabinoid release profile can be non-sigmoidal. The cannabinoid release profile can be linear. The cannabinoid release profile can be non-linear. The cannabinoid release profile can be instant release. The cannabinoid release profile can be non-instant release. The cannabinoid release profile can be delayed release. The cannabinoid release profile can be constant or sustained release. The cannabinoid release profile can be non-constant or non-sustained release.

Tablets can be formulated in sustained release format. Methods of making sustained release tablets are known in the art; see, for example, U.S. Patent Publication No. 2006/0051416 and U.S. Patent Publication No. 2007/0065512. Gradual-release tablets are known in the art; examples of such tablets are set forth in U.S. Patent No. 3,456,049, for example. A slow- or sustained-release form may delay disintegration or absorption of the composition or one or more components thereof.

In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 1 hour of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 2 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 3 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 4 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 5 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 6 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 7 hours of administration to a subject. In some cases, no more than 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 8 hours of administration to a subject.

A release profile is the relationship between time and the amount of a compound released into a subject or the concentration of the compound within the subject (e.g., within the plasma). Release profiles can be measured in a similar manner to plasma half-life. A composition can be administered to a subject, and samples (e.g., plasma samples or blood samples) can be taken from the subject at multiple time points. The concentration of the compound or compounds of interest can be measured in those samples, and a release profile can be plotted.

Compounds taken up into a subject via the gastrointestinal system can be transported to the liver before entering general circulation. Compounds susceptible to metabolic degradation in the liver can have their activities substantially reduced by the first-pass metabolism through the liver. Encapsulation (e.g., microencapsulation) of compounds can reduce first-pass metabolism of the compounds in the liver. Compositions in encapsulated form can be characterized by a first pass cannabinoid metabolism in a subject of at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than that of a non-encapsulated cannabinoid composition. Compositions in encapsulated form can be characterized by a cannabinoid excretion rate from a subject of at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than that of a non-encapsulated cannabinoid composition.

The compositions described herein, when administered to a subject, can have improved bioavailability, bioactivity, or both. Bioavailability is the fraction of an administered dosage of unchanged compound that reaches systemic circulation. Cannabinoid compositions in encapsulated form can be characterized by a bioavailability in a subject of at least 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 6.5, 7.0, 7.5, 8.0, 8.5, 9.0, 9.5, or 10.0 times that of a non-encapsulated cannabinoid composition. Cannabinoid compositions in encapsulated form can be characterized by a bioavailability in a subject of at least 5%, 10%, 15%, 20%, 25%, 30%, 35%, 40%, 45%, 50%, 55%, 60%, 65%, 70%, 75%, 80%, 85%, 90%, 95%, 96%, 97%,%, 98%, 99%, or 100%. Bioactivity, or biological activity, is the activity exerted by the active ingredient or ingredients in a composition. Cannabinoid compositions in encapsulated form can be characterized by a bioactivity in a subject of at least 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 6.5, 7.0, 7.5, 8.0, 8.5, 9.0, 9.5, or 10.0 times that of a non-encapsulated cannabinoid composition.

The compositions described herein can be used to treat a subject with a disease including but not limited to the conditions described in paragraph [0064]. The compositions described herein can be used to treat a subject with an injury including but not limited to the conditions described in paragraph [0064].

To treat a disease as used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

The methods disclosed herein can yield a synergistic effect. Use of the cannabinoid composition with the stem cell can synergistically enhance the function of the stem cell. In some instances the stem cell treatment and cannabinoid composition can be administered at sub therapeutic amounts. In some embodiments a sub-therapeutic amount of the stem cells and/or the cannabinoid composition can be used. A sub-therapeutic amount of an agent or therapy is an amount less than the effective amount for that agent or therapy, but when combined with an effective or sub-therapeutic amount of another agent or therapy can produce a result desired by the physician, due to, for example, synergy in the resulting efficacious effects, or reduced side effects.

A "synergistically effective therapeutic amount" or "synergistically effective amount" of an agent or therapy is an amount which, when combined with an effective or sub-therapeutic amount of another agent or therapy, produces a greater effect than when either of the two agents are used alone. In some embodiments, a synergistically effective therapeutic amount of an agent or therapy produces a greater effect when used in combination than the additive effects of each of the two agents or therapies when used alone. In certain preferred embodiments of this disclosure the stem cell therapy and cannabinoid composition exhibit a synergistic effect.

In some cases, efficacy can be evaluated by determining changes in overall survival, improvements in disease associated symptoms, disease-free survival, progression free survival, and/or time to treatment failure. Efficacy can be determined by various non-limiting methods, including monitoring changes in gene expression levels and biomarker expression levels.

### EXAMPLES

### Example 1 - Microencapsulated Cannabinoids improve stem cell engraftment

A hemp oil composition is produced, comprising cannabinoids including cannabidiol. The composition is microencapsulated via a microfluidic nozzle device. The microcapsules are administered to a subject prior to the subject receiving a stem cell treatment. Subsequent to the subject receiving the stem cell treatment the subject has increased engraftment of the stem cells compared to a control subject who did not receive microcapsules containing cannabinoids.

### Example 2 - Microencapsulated Cannabinoids improve stem cell survival during transplant

A hemp oil composition is produced, comprising cannabinoids including cannabidiol. The composition is microencapsulated via a microfluidic nozzle device. The microcapsules are administered to a subject prior to the subject receiving a stem cell treatment. Subsequent to the subject receiving the stem cell treatment the subject has more surviving stem cells compared to a control subject who did not receive microcapsules containing cannabinoids.

### Example 3 - Microencapsulated Cannabinoids reduce immune activation against transplanted stem cells

A hemp oil composition is produced, comprising cannabinoids including cannabidiol. The composition is microencapsulated via a microfluidic nozzle device. The microcapsules are administered to a subject concurrent with the subject receiving a bone marrow transplant. Subsequently, reduced graft rejection is seen compared to control subjects who did not receive microcapsules containing cannabinoids. Graft rejection is determined by using PCR amplification of variable number tandem repeats (VNTR) loci to identify donor and recipient cells in the bone marrow.

### Example 4 -Cannabinoids affect gene expression in mouse embryonic stem cells

Mouse embryonic stem cells are cultured in media treated with either cannabidiol (1 µM, 5 µM, 10 µM or 50 µM) or vehicle control. Samples are collected at days 0, 1, 2, 3, 4, 5, 6, 7, 8, 9, and 10 and RNA is extracted for next generation sequencing. Expression analysis is performed on the sequencing data to determine genes which are differentially expressed in the cells which are treated with cannabidiol. Differential expression of stem cell markers, differentiation markers, DNA replication, cell cycle, proliferation, apoptosis and cannabinoid pathway genes may be of particular interest.

### Example 5 -Cannabinoids affect sphere formation in mouse embryonic stem cells

Mouse embryonic stem cells are cultured in media treated with either cannabidiol (1 µM, 5 µM, 10 µM or 50 µM) or vehicle control for 10 days. After 10 days of culture the cells are transferred to a sphere formation assay. Each treatment group is scored for number of spheres formed, size and shape of spheres formed, cell types found in each sphere and the number of times the spheres can be passaged. The stem cells treated with cannabidiol may form spheres with higher efficacy, may form spheres with more neural type cells than the spheres formed by the vehicle treated cells, or may form spheres with more or less different types of cells compared to the vehicle treated cells.

### Example 6 -Cannabinoids increase engraftment of mouse hematopoietic stem cells delivered by tail vein injection

Mouse hematopoietic stem cells are isolated from bone marrow of a mouse expressing GFP under the control of a constitutive promoter. Recipient mice are prepared by exposing congenic mice to radiation sufficient to deplete the population hematopoietic stem cells. The isolated hematopoietic stem cells are delivered to the recipient mice by tail vein injection. Following the injection the mice are administered a control diet or a diet supplemented with a microencapsulated hemp oil composition comprising cannabinoids including cannabidiol. Blood samples are taken from both groups of mice at 4, 8, 12, 16, 20 24, 28 and 32 weeks and the number of donor derived cells is determined by screening cells for GFP expression.

An additional experiment is performed using the above protocol to determine the efficacy of hematopoietic stem cell transplant from mice which have been genetically engineered to lack cannabinoid receptors (CB null) into CB null recipient mice.

### Example 7 -Cannabinoids potentiate stem cell treatment after spinal cord injury

Mouse neural stem cells expressing green fluorescent protein (GFP) under the control of a constitutive promoter are treated with either cannabidiol (1 µM, 5 µM, 10 µM or 50 µM) or vehicle control for 10 days. The cells are then transplanted directly into the spinal cords of mice with spinal cord injuries. Mice are maintained on a control diet or a diet supplemented with a microencapsulated hemp oil composition comprising cannabinoids, including cannabidiol, at concentrations similar to those used in the cell culture media. Mice are monitored daily for signs of spinal cord recovery evaluations of locomotor function based on Basso Mouse Scale (BMS) scores, a locomotor rating scale based on the frequency analyses of seven locomotor categories, and rotarod treadmill tests. Two months after the stem cell treatment the mice are sacrificed and the spinal cords are histologically examined. The mice which receive the stem cells with cannabinoid treatment and food with cannabinoid supplement show more rapid and more robust recovery.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.
The following numbered clauses, describing aspects of the invention, are part of the description.
1. A method for potentiating function of a stem cell in a subject, comprising:
   (a) administering to said subject said stem cell;
   (b) administering to said subject a pharmaceutical composition comprising an encapsulated cannabinoid compound, wherein (b) takes place prior to, concurrent with, or subsequent to step (a).
2. The method of clause 1 wherein step (b) further comprises at least one terpene compound.
3. The method of clause 1 wherein treatment with step (b) reduces the side effects usually associated with step (a).
4. The method of clause 1 wherein said subject suffers or is suspected of suffering from a disease selected from the group of acute leukemia, chronic leukemia, and lymphoma, an inherited platelet abnormality, a plasma cell disorder, an autoimmune disease, a myeloproliferative disorder, a lymphoproliferative disorder, a phagocyte disorder, myelodysplastic syndrome, a histiocytic disorder, a congenital immune system disorder, Parkinson's disease, Amyotrophic lateral sclerosis, Alzheimer's disease, and multiple sclerosis, stroke, diabetes, infertility, vision loss or other eye diseases, a lysosomal storage disease, peripheral arterial diseases, ischemic limb injury, diabetes, heart disease, liver disease, bone disease, muscular dystrophy, dental disease, and cancer.
5. The method of clause 1 wherein said subject suffers from an injury selected from the group of spine and spinal cord injuries, wounds, thermal or chemical burns, sports injuries, occupational injuries, or a brain injury.
6. The method of clause 1 wherein said stem cell is from stem cells that are induced pluripotent stem cells, embryonic stem cells, fetal stem cells, or adult stem cells.
7. The method of clause 1 wherein said stem cell expresses at least one of AA4, AA4.1, P-gp (CD243), ABCB5, ABCG2 (CDw338), ALDH, alkaline phosphatase, alpha6-integrin, WNT2B, antithrombin III (AT), asialo GM1, Bcl-2, beta1-integrin, bromodeoxyuridine, c-kit (CD117), c-Met, C1qR(p), END (CD105), PROM1 (CD133) , ALCAM (CD166), ITGB1 (CD29), TNFRSF8 (CD30), PECAM-1 (CD31), Siglec-3 (CD33), CD34, CD44, NCAM (CD56), CD73, CD9, CD90, CDCP1, Circulating anticoagulants protein C (PC), CK19, CLV3, cyclic CMP, ECMA-7, EDR1, EEC, FGF-4, Flk-2, Flk1(+), Flt3/Flk2, FMS (CD115), FORSE-1, G alpha16, GDF3, GFPM, Gli2, Gli3, glial fibrillary acidic protein, glycoprotein IB, GSTA1, HAS2 gene expression, Her5, hMYADM, HSA, hsp25, Id2, IL-3Ralpha, Integrins, interleukin-3 receptor alpha chain, Iron oxide nanoparticles, KDR, Keratin 15 (aka. CK15, Cytokeratin 15) , Keratin 19 (aka. CK19, Cytokeratin 19, K19), Kit, L-selectin (CD62L), Lamin A/C, Lewis X antigen (Le(X)), LeX, Lgr5, Lrp4, MCM2, MCSP, Metallothionein (MT) crypt-restricted immunopositivity indices (MTCRII), monosomy 7, Mouse orthologue of ARX, MRP4, Msi-1, Musashi, Musashi-1, Mutant BCRP, nestin, neurofilament microtubule-associated protein 2, neuron-glial antigen 2 (NG2), notch 1, nrp-1, Nucleostemin, OC.3, Oct-4, OST-PTP, P-gp/MDR1, p21, p63, p75, PCLP, PCNA, PECAM, PgP-1, phosphorylating-p38, Podocalyxin, procalcitonin (PCT), PSCs, pSV2gpt, PTPRC, purified LRC, Rat liver fatty acid-binding protein/human growth hormone transgenes (Fabpl/hGH), RC1 antigen, Rex-1, Sca-1, SCF, Sialyl-lactotetra, Side Population (SP), SOX10, SOX2, SOX9, SP phenotype, SSEA-1, SSEA-3, SSEA-4, Stat3, Stat5, Stella, Stra8, Stro-1, Tartrate-resistant acid phosphatase (TRAcP), TdT, telomerase reverse transcriptase, electrophoretic pattern of hemoglobin, Thrombomucin, Thy-1, Tra-1-60, TWIST1, VEGFR-2, vimentin, X-Smoothened, XKrk1, or Zac1.
8. The method of clause 1 wherein said stem cell is from stem cells that exhibit totipotency, pluripotency, bipotency or monopotency.
9. The method of clause 1 wherein said stem cell is from stem cells that yield fibroblasts, keratinocytes, melanocytes, cold-sensitive primary sensory neuron, auditory inner hair cell or organ of Corti, Merkel cell, Photoreceptor cells, taste bud cell, cholinergic neural cells, adrenergic neural cells, peptidergic neural cells, hepatocytes, adipocytes, liver lipocytes, kidney glomerulus podocytes, pancreatic duct cell, gall bladder epithelial cells, pericytes, corneal fibroblasts, skeletal muscle cells, cardiac muscle cells, Purkinje fiber cells, erythrocytes, megakaryocytes, monocytes, Langerhans cells, osteoclasts, osteoblasts, dendritic cells, microglial cells, neutrophil granulocyte, hybridoma cells, mast cells, helper T cells, suppressor T cells, cytotoxic T cells, natural killer T cells, B cells, oocytes, spermatids, ovarian follicle cells, Schwann cells, satellite glial cells, enteric glial cells, astrocytes, neuron cells, oligodendrocytes, anterior lens epithelial cells, crystallin-containing lens fiber cells, somatotropes, Corticotropes, melanotropes, thyroid gland cells, or odontocytes.
10. The method of clause 1 wherein said stem cell is from stem cells that are delivered via intravenous infusion, intradermal, transplanted microvascular bed of bone marrow, subcutaneous, oral (e.g., ingestion or inhalation), transdermal (topical), transmucosal, rectal administration, engineered monolayer tissue transplantation, intraarterially, intramuscularly, intratracheally, intraperitoneally, intravitreally, or via direct injection to a target site.
11. The method of clause 1 wherein steps (a) and (b) exhibit a synergetic effect on a process in said subject.
12. The method of clause 1, wherein said pharmaceutical composition comprises nanocapsules, wherein said nanocapsules comprise an individual nanocapsule comprising said encapsulated cannabinoid compound.
13. The method of clause 12, wherein said nanocapsules increase stem cell growth.
14. The method of clause 12, wherein said nanocapsules are administered following said stem cell therapy.
15. The method of clause 12, wherein said nanocapsules are administered to said subject by inhalation.
16. The method of clause 12, wherein said nanocapsules are vaporized.
17. The method of clause 12, wherein said nanocapsules are nebulized.
18. The method of clause 12, wherein said nanocapsules are administered orally.
19. The method of clause 12, wherein said nanocapsules are incorporated into a food or beverage.
20. The method of clause 12, wherein said nanocapsules are administered topically.
21. The method of clause 12, wherein said nanocapsules are water soluble.
22. The method of clause 2, wherein said at least one terpene compound is derived from quillaja *(Quillaja saponaria).*
23. The method of clause 1, wherein said cannabinoid compound comprises cannabidiol (CBD).
24. The method of clause 1, wherein said cannabinoid compound comprises 0.3% tetrahydrocannabinol (THC) or less.
25. The method of clause 1, wherein said subject suffers or is suspected of suffering from a disease or injury, and wherein, subsequent to (b), said subject is monitored for a progress of said disease or injury in response to said subject being administered said pharmaceutical composition.
26. The method of clause 25, wherein said subject suffers or is suspected of suffering from said disease, and wherein, subsequent to (b), said subject is monitored for a progression or regression of said disease in response to said subject being administered said pharmaceutical composition.
27. A kit comprising:
   (a) a composition comprising an effective amount of an encapsulated cannabinoid compound; and
   (b) instructions for administering to a subject, undergoing a stem cell therapy, a therapeutically effective amount of said food composition.
28. The kit of clause 27, wherein said composition is a food composition.
29. The kit of clause 27, wherein said composition is a pharmaceutical composition.
30. The kit of clause 27, wherein said composition comprises nanocapsules, wherein said nanocapsules comprise an individual nanocapsule comprising said encapsulated cannabinoid compound.
31. The kit of clause 30, wherein said nanocapsule delivery device is a nebulizer.
32. The kit of clause 30, wherein said nanocapsule delivery device is a vaporizer.
33. The kit of clause 30, wherein said nanocapules include a terpene compound.
34. The kit of clause 33, wherein said terpene compound is derived from quillaja (*Quillaja saponaria*)*.*

## Claims

1. A composition for use in potentiating function of a stem cell in a subject undergoing stem cell therapy comprising:
a) an encapsulated cannabinoid compound; and
b) a terpene compound derived from quillaja *(Quillaja saponaria).*

2. The composition for use according to claim 1, wherein said encapsulated cannabinoid compound is encapsulated in nanocapsules.

3. The composition for use according to claim 1, wherein the nanocapsules are vaporized.

4. The composition for use according to claim 1, wherein the nanocapsules are nebulized.

5. The composition for use according to claim 1, wherein the nanocapsules are administered orally.

6. The composition for use according to claim 1, wherein the nanocapsules are incorporated into a food or beverage.

7. The composition for use according to claim 1, wherein the nanocapsules are administered topically.

8. The composition for use according to claim 1, wherein the cannabinoid compound comprises cannabidiol.

9. The composition for use according to claim 1, wherein said potentiating function of a stem cell comprises increasing survival of the stem cell in the subject, increasing engraftment of the stem cell into a desired niche in the subject, increasing the migration potential of the stem cell in the subject, increasing the differentiation of the stem cell, or prolonging the life of the stem cell in the subject.

10. The composition for use according to claim 1, wherein the subject is undergoing stem cell therapy for a spinal cord injury.

11. The composition for use according to claim 1, wherein the cannabinoid release profile is sigmoidal.
